(19) Europäisches Patentamt
European Patent Office
Office européen des brevets

(11) Publication number: **0 291 245 B1**

(12) **EUROPEAN PATENT SPECIFICATION**

(45) Date of publication of patent specification: **22.07.92**

(51) Int. Cl.5: **C07C 233/67**, C07C 313/00, A61K 31/165, A61K 7/48

(21) Application number: **88304120.4**

(22) Date of filing: **06.05.88**

The file contains technical information submitted after the application was filed and not included in this specification

(54) **Benzoylaminophenoxybutanoic acid derivatives.**

(30) Priority: **11.05.87 JP 112618/87**
**17.09.87 JP 231194/87**

(43) Date of publication of application:
**17.11.88 Bulletin 88/46**

(45) Publication of the grant of the patent:
**22.07.92 Bulletin 92/30**

(84) Designated Contracting States:
**AT BE CH DE ES FR GB GR IT LI LU NL SE**

(56) References cited:
**EP-A- 0 173 516**
**GB-A- 1 088 295**
**US-A- 3 398 187**

**PATENT ABSTRACTS OF JAPAN, vol. 9, no. 309 (C-318)(2032), 5 December 1985; & JP-A-60 146 855**

(73) Proprietor: **ONO PHARMACEUTICAL CO., LTD.**
**14, Doshomachi 2-chome Higashi-ku**
**Osaka 541(JP)**

(72) Inventor: **Nakai, Hisao**
**13-8 Nishikammuri 2**
**Takatsuki-city Osaka(JP)**
Inventor: **Terashima, Hiroshi**
**20-17, Okutenjincho 2**
**Takatsuki-city Osaka(JP)**
Inventor: **Arai, Yoshinobu**
**8-505, Wakayamadai 1-5**
**Shimamotocho Mishima-gun Osaka(JP)**

(74) Representative: **Bentham, Stephen et al**
**J.A. Kemp & Co. 14 South Square Gray's Inn**
**London WC1R 5EU(GB)**

EP 0 291 245 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to novel benzoylaminophenoxybutanoic acid derivatives, to processes for their preparation, pharmaceutical compositions containing them and their use.

Many theories have been expounded concerning the origin of androgenic alopecia based on, for example, (1) an imbalance of hormones, (2) genetic factors, (3) circulatory failure or (4) nutrition. It has also been suggested that testosterone (androgenic hormone) played an important role in the generation of hair.

The theory of Adachi et al (Biochem. Biophys. Res. Commun., 41, 884 (1970)) in which the relation between testosterone and androgenic alopecia is proved by biochemical experiments, is as follows:

i) first, testosterone biosynthesized in the testis is converted into dihydrotestosterone by 5α-reductase present in, inter alia, hair follicles and sebaceous glands on the head;

ii) the dihydrotestosterone causes a substantial reduction in the activities of adenyl cyclase;

iii) the level of cyclic-AMP in cells decreases; and

iv) a lowering of energy generation of hairs and limbus and suppression of protein synthesis is induced.

According to this theory it is thought that, as a result of the series of phenomena just described, hairs in the growing phase shift to the resting phase, the terminal hairs change to soft hairs, and androgenic alopecia results.

A report by H.V. Schweikert (J. Clin. Endocr., 38, 811 (1974)) supports this theory in that large quantities of metabolites produced by 5α-reductase such as dihydrotestosterone are present in hair follicles of androgenic alopecia patients in quantities greater than those in females or healthy males.

It has also been reported that dihydrotestosterone produced from testosterone by 5α-reductase also plays an important physiological role in the generation of acnes (acne, pimples etc.) in addition to androgenic alopecia. J.B. Hay et al (Br. J. Dermatol., 91, 123 (1974)) has reported from a comparative study of the affected skin of acne-patients and healthy skin that the metabolism of testosterone by 5α-reductase was enhanced in the parts affected by aggravated acne.

G. Sansone et al (See J. Invest. Dermatol., 56, 366 (1971) found that the ability to synthesize dihydrotestosterone from testosterone increased from two to twenty times in the affected part of acne-patients compared to that in healthy man, and suggested that dihydrotestosterone generated by 5α-reductase is strongly related to the generation or aggravation of acne.

Dihydrotestosterone is also related to hypertrophy of the prostate. Cowan et al (J. Steroid Biochemistry, 11, 609 (1979)) reported that much dihydrotestosterone existed in the prostate of prostatic hypertrophy patients. It has been reported (See J. Clinical Endocrinol. and Metabolism, 56, 139 (1983)) that the activity of 5α-reductase in the prostate of prostatic hypertrophy patients is abnormally increased.

It has become clear from these reports that dihydrotestosterone plays an important role in the generation and development of prostatic hypertrophy.

Research has been carried out to discover compounds active as 5α-reductase inhibitors: the compounds have been mainly steroids or derivatives thereof.

Widespread investigations have been carried out in order to discover compounds which have a non-steroidal structure and possess inhibitory activity on 5α-reductase. We have filed a number of applications directed to compounds wherein cinnamic acid or benzoic acids form amides with anilines, [See Japanese Patent Kokai Nos. 60-97946, 60-116657, 60-142936, 60-142941, 60-146855, 61-126061 (equivalent to EP 0173516), 62-198652 and 62-198653].

For example, EP 0173516 and Japanese Patent Kokai No. 61-126061 describe a very wide range of amide compounds which possess inhibitory activity on 5α-reductase. The compounds most closely related to those of the present invention, and which are described as possessing antagonistic activity on leukotrienes, inhibitory activity on 5α-reductase, on phospholipase and on aldose reductase are defined by the general formulae:

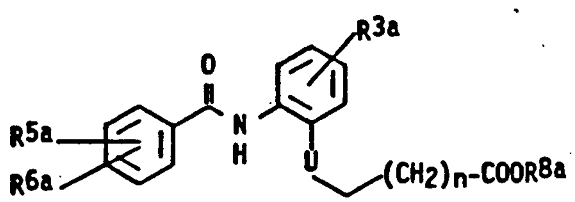

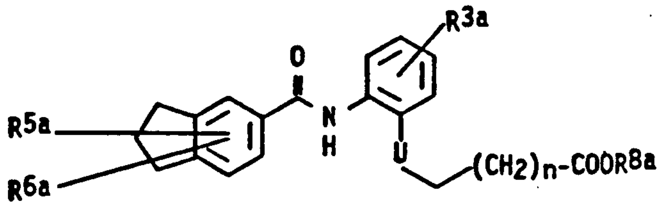

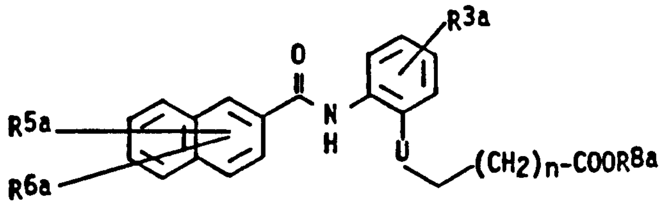

[wherein, $R^{3a}$ represents, inter alia, a hydrogen atom or halogen atom, $R^{5a}$ and $R^{6a}$ represent, independently, a hydrogen or halogen atom, or a straight- or branched-chain alkyl, alkenyl or alkynyl group of from 1 to 20 carbon atom(s) in which from 1 to 5 carbon atoms may be replaced by an oxygen atom, sulfur atom, halogen atom, nitrogen atom, benzene ring, thiophene ring, napthalene ring, carbocyclic of from 4 to 7 carbon atoms, carbonyl group, carbonyloxy group, hydroxy group, carboxy group, azido group, or nitro group,
$R^{8a}$ represents a hydrogen atom or a straight- or branched-chain alkyl group of from 1 to 6 carbon atom(s),
U represents an oxygen atom or sulfur atom, and
n represents an integer of from 1 to 10].

In the first general formula depicted above when one of $R^{5a}$ and $R^{6a}$ represent a phenylalkoxy group which may be substituted (i.e an alkyl group of one carbon atom carrying an optionally substituted benzene ring), the group will correspond to the $R^2$ group in the compounds of the present invention depicted hereinafter. In this case the other of $R^{5a}$ and $R^{6a}$ remains and may represent hydrogen or a single substituent.

The compounds of the present invention, depicted hereinafter, are distinguished from those just described by, inter alia, the presence of two substitutents, corresponding to the single substitutent represented by $R^{5a}$ and $R^{6a}$, on specific adjacent positions of the benzene ring: the two substituents may also represent, together with the carbon atoms to which they are attached, a cyclopentane, cyclohexane or benzene ring.

Benzoylamino-phenoxy (and phenylthio) butanoic acids are also described in
British Patent No. 1077936,
British Patent No. 1088295,
United States Patent No. 3549689, and
PCT Patent Publication No. 8605779.

However, all of the compounds described are distinguished from those of the present invention both in chemical structure and in their biological activity. Whereas the compounds of the present invention possess inhibitory activity on 5α-reductase the known compounds are described as having anti-inflammatory activity (including antagonistic activity on SRS).

British Patent No. 1077936 describes inter alia, compounds embraced by the general formula:

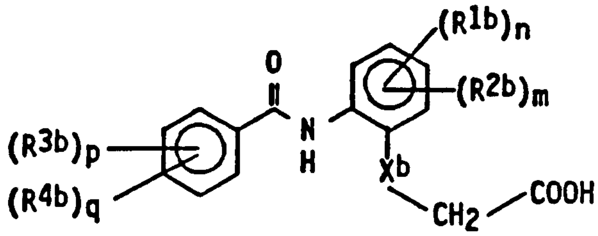

(wherein $R^{1b}$-$R^{4b}$ each represents alkyl or alkoxy of from 1 to 4 carbon atom(s), hydroxy, halogen, perhaloalkyl of from 1 to 4 carbon atom(s), nitro, or alkylsulfonyl of from 1 to 4 carbon atom(s),
$X^b$ represents, oxygen or sulfur, and
m, n, p and q each represents zero or one).

British Patent No. 1088295 describes, inter alia, compounds embraced by the general formula:

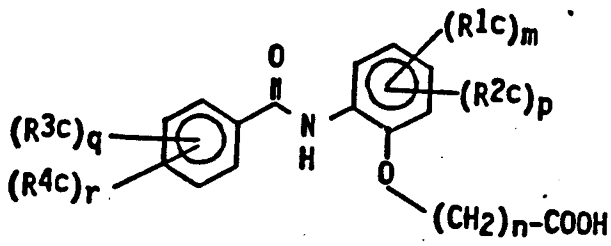

(wherein $R^{1c}$-$R^{4c}$ each represent alkyl or alkoxy of from 1 to 4 carbon atom(s), trifluoromethyl or halogen,
n represents 2 or 3, and
m, p, q and r each represents 0 or 1).

US Patent No. 3549689 describes, inter alia, compounds embraced by the general formula:

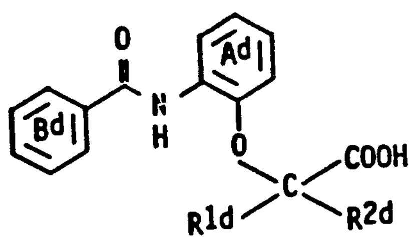

(wherein $R^{1d}$ represents hydrogen or methyl,
$R^{2d}$ represents methyl or ethyl,
the $A^d$ ring may be substituted by, inter alia, methyl or halogen, and
the $B^d$ ring may be substituted by one or two substituents selected from nitro, trifluoromethyl, chlorine, fluorine and alkyl or alkoxy of from 1 to 4 carbon atom(s)).

The substituents $R^{3b}$, $R^{3c}$, and $R^{4b}$ and $R^{4c}$, and the substituent(s) on the $B^d$ ring, in the general formula just depicted all distinguish known compounds from those of the present invention.

PCT Patent Publication No. 8605779 describes, inter alia, compounds embraced by the general formula:

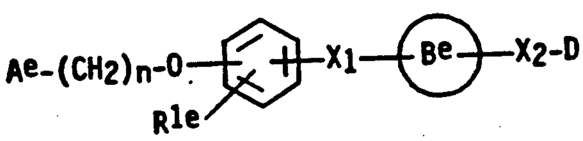

[wherein, $A^e$ represents hydrogen, phenyl or phenoxy,
n represents 3 - 10,
$R^{1e}$ represents hydrogen or lower alkoxy,
$X^1$ represents -CONH-,

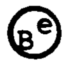 represents 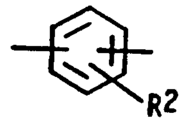

(wherein $R^2$ represents hydrogen, halogen or nitro).
$X^2$ represents -0-$Y^4$
(wherein $Y^4$ represents alkylene of from 1 to 6 carbon atom(s)), and
D represents carboxy or alkoxycarbonyl].

The compounds of the present invention, depicted hereinafter, are distinguished from those disclosed in PCT Patent Publication No. 8605779 by, inter alia, the replacement of the single substituent $R^{1e}$, which represents hydrogen or an alkoxy substituent on any of the free positions of the benzene ring, by two identical substituents (not alkoxy) at specific adjacent positions on the ring : the two substituents may also represent, together with the carbon atoms to which they are attached, a cyclopentane, cyclohexane or benzene ring.

The present invention accordingly provides a benzoylaminophenoxybutanoic acid derivative of the general formula:

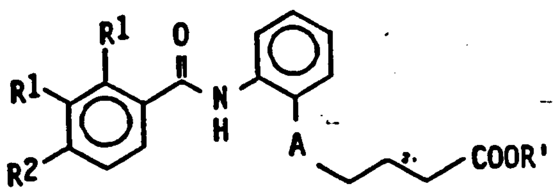 (I)

[wherein R′ represents a hydrogen atom or an alkyl group of from 1 to 4 carbon atom(s),
A represents an oxygen atom, sulfur atom or sulfinyl (SO) group,
the two groups $R^1$ simultaneously represent methyl groups or chlorine atoms or form, together with the carbon atoms of the benzene ring to which they are attached, a cyclopentane, cyclohexane or benzene ring,
$R^2$ represents a group of the general formula:

(i)

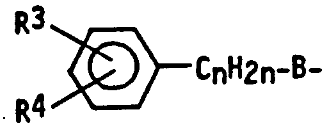

(ii)

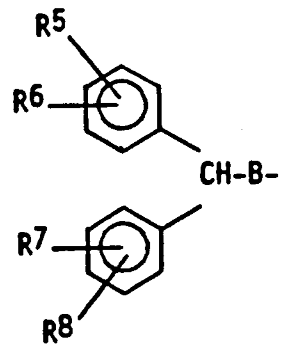

or

(iii)

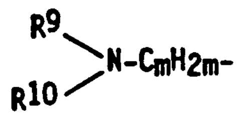

{wherein B represents an oxygen atom, a sulfur atom or a group of the general formula $-NR^{11}$ (wherein $R^{11}$ represents a hydrogen atom or an alkyl group of from 1 to 4 carbon atom(s)),
$R^3$, $R^4$, $R^5$, $R^6$, $R^7$ and $R^8$ represent, independently, a hydrogen atom, an alkyl group of from 1 to 4 carbon atom(s), a halogen atom, a trifluoromethyl group or a cyclobutylmethyl group,
m represents 0 or 1,
n represents an integer of from 1 to 5, and
$R^9$ and $R^{10}$, which may be the same or different, each represents a hydrogen atom, an alkyl group of from 1 to 5 carbon atom(s) or a group of the general formula:

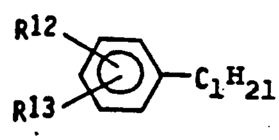

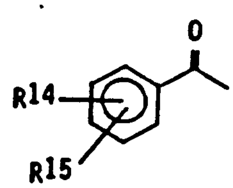

or

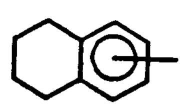

(wherein $R^{12}$, $R^{13}$, $R^{14}$ and $R^{15}$ represent, independently, a hydrogen atom, an alkyl group of from 1 to 4 carbon atom(s), a halogen atom, a trifluoromethyl group or a cyclobutylmethyl group, and 1 represents an integer of from 1 to 4),
with the proviso that $R^9$ and $R^{10}$ do not simultaneously represent hydrogen atoms},
and non-toxic salts thereof.

In this specification and the accompanying claims all isomers are included unless otherwise specified. For example, alkyl or alkylene groups include straight and branched ones, and the present invention includes isomers arising from the existence of asymmetric carbon atoms in, for example, branched alkyl groups.

In general formula (I), alkyl groups of from 1 to 4 carbon atom(s) represented by R′, $R^3$-$R^8$, and $R^{11}$-$R^{15}$ are methyl, ethyl, propyl and butyl groups and isomers thereof.

In general formula (I), alkyl groups of from 1 to 5 carbon atom(s) represented by $R^9$ and $R^{10}$ are the alkyl groups just described above, the pentyl group and isomers thereof.

In general formula (I), halogen atoms represented by $R^3$-$R^8$, and $R^{11}$-$R^{15}$ are fluorine, chlorine, bromine and iodine atoms.

In $R^2$ of general formula (I), the grouping $-C_nH_{2n}-$ is a straight or branched chain alkylene group of from 1 to 4 carbon atom(s), specifically methylene, ethylene, trimethylene and tetramethylene groups and isomers thereof.

In $R^2$ of general formula (I), the grouping $-C_lH_{2l}-$ is a straight or branched chain alkylene group of from 1 to 5 carbon atom(s), specifically the alkylene groups just described above, the pentamethylene group and isomers thereof.

In $R^2$ of general formula (I), the grouping $-C_mH_{2m}-$ represents a single bond or a methylene group.

The compounds of general formula (I) may be converted into the corresponding salts by known methods. Water soluble salts are preferable. Suitable salts include, for example,

- salts of alkali metals, e.g. sodium and potassium,
- salts of alkaline earth metals, e.g. calcium and magnesium,
- ammonium salts,
- salts of pharmaceutically acceptable amines, e.g. tetraalkylammonium salts such as tetramethylammonium, triethylamine, methylamine, dimethylamine, cyclopentylamine, benzylamine, phenethylamine, piperidine, monoethanolamine, diethanolamine, tris (hydroxymethyl)-aminomethane, lysine, arginine and N-methyl-D-glucamine salts.

According to a feature of the invention compounds of general formula (I) which conform to the general formula:

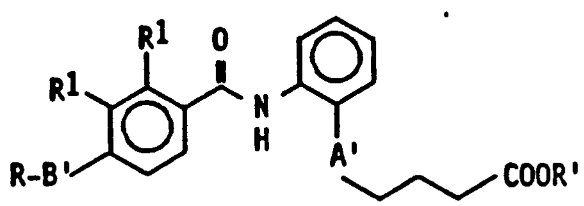

(Ia)

[wherein A′ represents an oxygen atom or sulfur atom,
R represents a group of general formula:

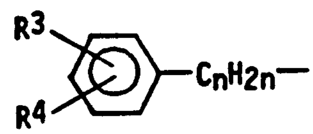

or

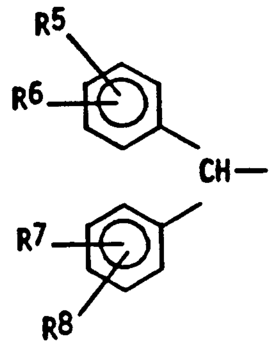

(wherein the various symbols are as hereinbefore defined), $B'$ represents an oxygen atom or a sulfur atom, and the other symbols are as hereinbefore defined] are prepared by a process which comprises reacting a carboxylic acid of the general formula:

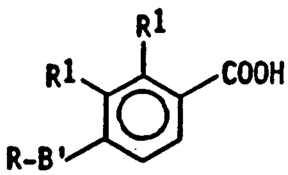

(II)

(wherein all of the symbols are as hereinbefore defined) or an activated derivative thereof with an amine of the general formula:

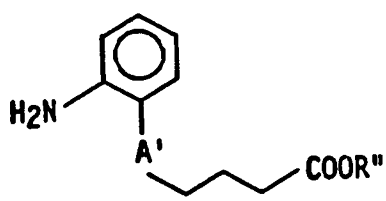

(III)

[wherein $R''$ represents an alkyl group of from 1 to 4 carbon atom(s) and the other symbols are as hereinbefore defined] and, if desired, subjecting to hydrolysis the resulting compound to convert the grouping $COOR''$ to a group COOH.

Methods for the preparation of an amide bond by reacting a carboxylic acid or an activated derivative thereof with an amine are known and include, for example,

(A) using a mixed acid anhydride as the activated derivative,

(B) using an acid halide as the activated derivative,

(C) using a condensing agent, e.g. dicyclohexylcarbodiimide (DCC).

Method (A) using a mixed acid anhydride may be carried out, for example, by reacting, a mixed acid anhydride obtained by acylating a carboxylic acid of the general formula (II) with an acyl halide (e.g. pivaloyl chloride, tosyl chloride or mesyl chloride) or an acid derivative (e.g. ethyl chloroformate or isobutyl chloroformate) in the presence of a tertiary amine (e.g. pyridine, triethylamine or picoline) in an inert organic

solvent (e.g. chloroform, methylene chloride, diethyl ether or THF) or without solvent at from 0 ° C to 40 ° C, with an amine of the general formula (III) in an inert organic solvent (for example one of those described above) at from 0 ° C to 40 ° C.

Method (B) using acid halide may be carried out, for example, by reacting, an acid halide obtained by treating a carboxylic acid of the general formula (II) with an acid halide (e.g. thionyl chloride or oxalyl chloride) in an inert organic solvent (e.g. as described above) at from -20 ° C to the reflux temperature of the solvent used, with an amine of the general formula (III) optionally in the presence of a tertiary amine (e.g. as described above) in an inert organic solvent (e.g. as described above) at from 0 ° C to 40 ° C.

Method (C) using a condensing agent such as DCC may be carried out, for example, by reacting a carboxylic acid of the general formula (II) with an amine of the general formula (III), using a condensing agent such as DCC, in the presence or absence of tertiary amine (e.g as described above), in an inert organic solvent (e.g as described above) or without solvent, at from 0 ° C to 40 ° C. The reactions of (A), (B) and (C) are preferably carried out in an inert gas atmosphere (using e.g. argon or nitrogen) and under anhydrous conditions.

The hydrolysis to convert the group COOR″, to a group COOH may be carried out by known methods for example, using an aqueous solution of alkali (e.g. lithium hydroxide, potassium hydroxide, sodium hydroxide, potassium carbonate or sodium carbonate) in a water-miscible organic solvent (e.g dimethoxyethane, tetrahydrofuran (THF), dioxane, ethanol, methanol or DMSO). The reaction is carried out at a temperature of from -10 ° C to 100 ° C.

According to a further feature of the invention compounds of general formula (I), which conform to the general formula:

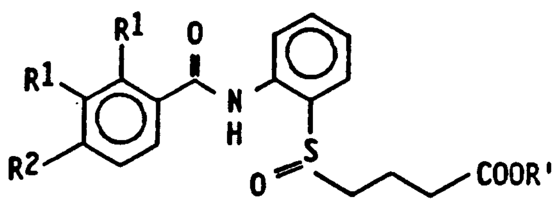

(Ib)

(wherein all of the symbols are as hereinbefore defined) are prepared by a process which comprises oxidizing a compound of general formula (I) wherein A is a sulfur atom, i.e. a compound of the general formula:

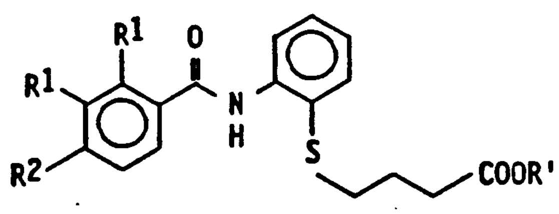

(Ib')

(wherein all of the symbols are as hereinbefore defined) to convert the group -S- to a group -S( = O)-.

The oxidation may be carried out by known methods, for example, using an aqueous solution of periodate (e.g. sodium periodate), in a water-miscible organic solvent (e.g. methanol, ethanol or THF), at a temperature of from 0 ° C to 50 ° C.

According to a further feature of the invention compounds of general formula (I) wherein A is an oxygen atom or a sulfur atom, B is a group of the general formula $NR^{11}$ (wherein $R^{11}$ is as hereinbefore defined) and $R^2$ represents a group of the general formula (iii), i.e. compounds of the general formula:

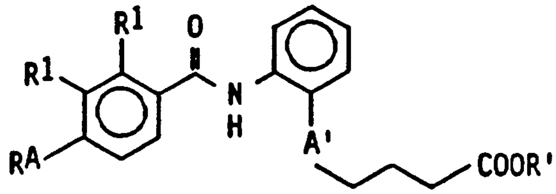

(Ic)

[wherein $R^A$ represents a group of the general formula:

(i')

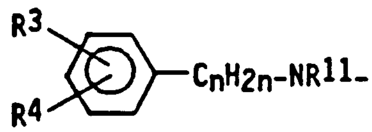

(ii')

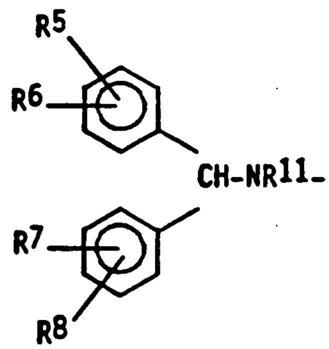

or

(iii')

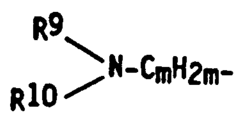

(wherein all of the symbols are as hereinbefore defined) and the other symbols are as hereinebfore defined] are prepared by the process which comprises reacting a compound of the general formula:

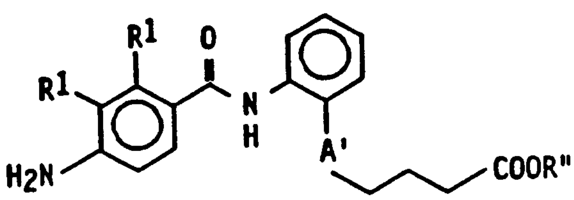

(IV)

[wherein all of the symbols are as hereinbefore defined] with a compound of the general formula:

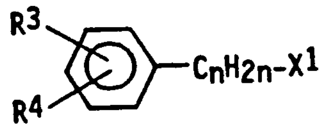

(V)

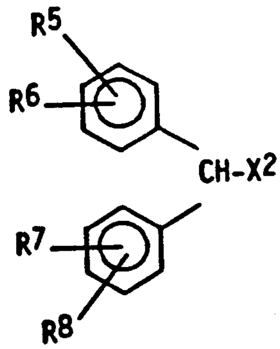

(VI)

$R^9$ - $X^3$ (VII)
$R^{10}$ - $X^4$ (VIII)

and/or

$R^{11}$ - $X^5$ (IX)

[wherein $X^1$, $X^2$, $X^3$, $X^4$ and $X^5$ each represent a halogen atom, a tosyl group or a mesyl group] and then, if desired, subjecting the resulting compound to hydrolysis to convert the group -COOR″ to a group -COOH, or by reacting a compound of the general formula:

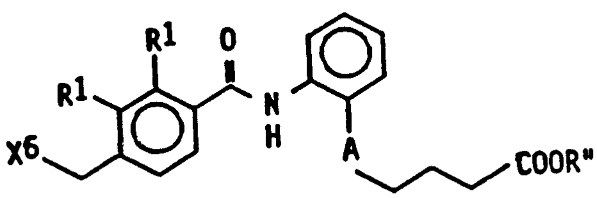

(X)

[wherein $X^5$ represents a halogen atom, a tosyl group or a mesyl group, and the other symbols are as hereinbefore defined] and a compound of the general formula:

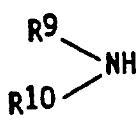

(XI)

[wherein $R^9$ and $R^{10}$ are as hereinbefore defined] and then, if desired, subjecting the resulting compound to hydrolysis to convert the group COOR″, to a group -COOH.

The hydrolysis may be carried out by known methods, for example as hereinbefore described. The N-alkylation reactions using compounds of the general formulae (V), (VI), (VII), (VIII) and (IX) and the reaction of compounds (X) and (XI) may also be carried out by known methods for example, using an aqueous or non-aqueous solution of alkali (e.g. potassium carbonate or sodium bicarbonate), in a water miscible organic solvent (e.g methyl ethyl ketone, acetonitrile, propanol or DMF).

Intermediates of the general formulae (II), (III), (IV) and (X) may be prepared as described in reaction scheme (A) which follows. The reactions in the scheme are known:

$X^7$ represents a halogen atom, a tosyl group or a mesyl group,
$R^{20}$ represents an alkyl group of from 1 to 4 carbon atom(s) and the other symbols are as hereinbefore defined.

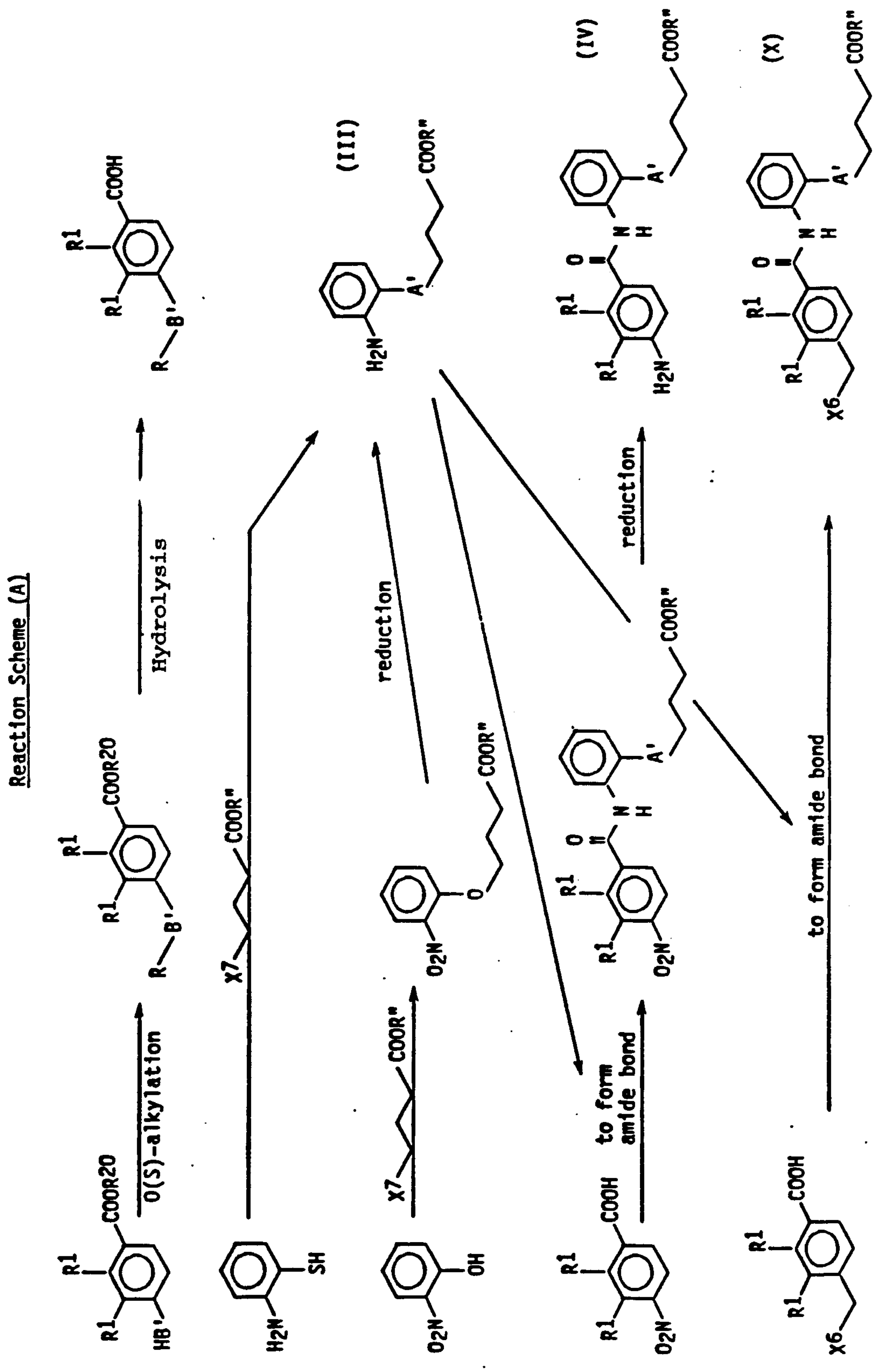


In the processes described in this specification, in each reaction, products may be purified by conventional methods, for example, distillation at atmospheric or reduced pressure, high performance liquid

chromatography, thin layer chromatography using silica gel or magnesium silicate or washing or recrystallization. Purification may be carried out after each reaction or after a series of reactions.

Starting materials and reagents for use in the processes of the present invention are known per se or may be prepared by known methods.

The compounds of general formula (I) possess an inhibitory activity on 5α-reductase and are therefore useful for the prevention and/or treatment of diseases resulting from the excess generation of dihydrotestosterone in mammals, especially humans. Such diseases include, for example, alopecia, e.g. androgenic alopecia, acnes and hypertrophy of the prostate.

The inhibitory activity on 5α-reductase of the compounds of the present invention is demonstrated by the screening system described hereafter.

Inhibitory activity on 5α-reductase in vitro

(1) Method

The test was carried out with reference to the method of J. Shimazaki et al [See Endocrinol., Japan., 18, 179 (1971)].

Male rat's prostate (4g) was homogenized with three times its volume of 0.1M HEPES buffer (pH 7.4) including 0.25M cane sugar and was then centrifuged at 3000 r.p.m. for 10 mins.

The precipitate was suspended in the buffer solution described above (10ml), and the suspension was centrifuged at 3000 r.p.m. for 5 mins. The resulting precipitate was suspended in the buffer solution (3ml) described above and was used as a source of enzyme.

A reaction mixture (total volume 0.1ml) of [4-$C^{14}$]-testosterone (1.5n mol, 1.5 x $10^5$ cpm), NADPH (0.5 μmol), enzyme source (0.03ml) prepared as described above and several concentrations of the compounds of the present invention was incubated for 60 mins at 37°C. Enzyme reaction was quenched by addition of a mixture (1.0ml) of diethyl ether, petroleum ether and acetic acid (80:20:1), and the mixture was centrifuged at 3000 r.p.m. for 5 mins. The supernatant (200 μl) was spotted on silica gel thin layer plate. The spot on the plate was developed with a mixture of chloroform, methanol and acetic acid (99:0.8 : 0.2). Radioactivity of the dihydrotestosterone on each plate was measured by radio-autography TLC scanning and an inhibitory ratio was calculated. The results are shown in the following Table 1.

Table I

| 5α-reductase inhibitory activity | | | |
|---|---|---|---|
| Example No. of the compound | $IC_{50}$ (μM) | Example No. of the compound | $IC_{50}$ (μM) |
| 2(a) | 2.1 | 2(j) | 2.5 |
| 2(b) | 0.38 | 2(k) | 2.6 |
| 2(c) | 1.3 | 2(l) | 1.0 |
| 2(d) | 0.98 | 2(m) | 0.75 |
| 2(e) | 1.4 | 2(n) | 0.95 |
| 2(f) | 0.27 | 2(o) | 0.005 |
| 2(g) | 0.34 | 2(p) | 0.011 |
| 2(h) | 0.26 | 2(q) | 0.2 |
| 2 | 0.12 | 3 | 0.70 |
| 2(i) | 2.0 | | |

The inhibitory activity on 5α-reductase of the compounds of the present invention render them useful for the prevention and/or treatment of disease resulting from excess generation of dihydrotestosterone in mammals, especially humans. Moreover, it was confirmed that the toxicity of the compounds of the present invention was very low so that they may be used as medicine with sufficient safety.

The compounds of the present invention will normally be administered systemically (mainly in the

prevention and/or treatment of prostatic hypertrophy) or partially (mainly in the prevention and/or treatment of alopecia and acne), usually by oral or parenteral administration.

The doses to be administered are determined depending upon, for example, age, body weight, symptoms, the desired therapeutic effect, the route of administration, and the duration of the treatment. In the human adult, for the treatment and/or prevention of prostatic hypertrophy, the doses per person per dose are generally from 1 mg to 1 g, by oral administration, up to several times per day, and from 100 µg to 100 mg, by parenteral administration (preferably intravenous administration) up to several times per day.

In the human adult, for the treatment and/or prevention of alopecia and/or acne, the doses per person per dose are generally from 10 µg to 50 mg, by dermal administration up to several times per day.

As mentioned above, the doses to be used depend upon various factors. Therefore, there are cases in which doses lower than or greater than the ranges specified above may be used.

The present invention provides pharmaceutical compositions which comprise a compound of general formula I or a non-toxic salt thereof in association with a pharmaceutically acceptable carrier or coating.

The compounds of the present invention may be administered as solid compositions, liquid compositions or other compositions by oral administration and injections, external compositions and, e.g. suppositories, for parenteral administration.

Solid compositions for oral administration, include compressed tablets, pills, dispersible powders, capsules and granules. In such compositions, one or more of the active compound(s) is or are, admixed with at least one inert diluent (e.g. lactose, mannitol, glucose, hydroxypropylcellulose, microcrystalline cellulose, starch, polyvinylpyrrolidone or magnesium metasilicate aluminate). The compositions may also comprise, as is normal practice, additional substances other than inert diluents for example, lubricating agents (e.g. magnesium stearate), disintegrating agents (e.g. cellulose calcium gluconate), and agents to assist dissolution (e.g. glutamic acid or aspartic acid) and stabilizing agents (e.g. lactose).

The tablets or pills may, if desired, be coated with gastric or enteric material (e.g. sugar, gelatin, hydroxypropylcellulose or hydroxypropylmethylcellulose phthalate).

Capsules may be soft or hard.

Liquid compositions for oral administration include pharmaceutically-acceptable emulsions, solutions, suspensions, syrups and elixirs.

In such compositions, one or more of the active compound(s) is or are dissolved or dispersed in inert diluent(s) (e.g. purified water or ethanol).

Besides inert diluents, such compositions may also comprise adjuvants (e.g. wetting agents or suspending agents), sweetening agents, flavouring agents, perfuming agents and preserving agents.

Other compositions for oral administration include spray compositions which may be prepared by known methods and which comprise one or more of the active compound(s).

Spray compositions may comprise additional substances other than inert diluents, e.g. stabilizing agents (e.g. sodium sulfite), isotonic buffer (using, e.g. sodium chloride, sodium citrate, citric acid).

For the preparation of such spray compositions, for example, the method described in United States Patent No. 2868691 or 3095355 may be used.

Injectable compositions for parenteral administration include sterile aqueous or non-aqueous solutions, suspensions and emulsions. In such compositions, one or more of the active compound(s) is or are admixed with at least one inert aqueous diluent(s) (e.g. distilled water for injection or physiological salt solution) or inert non-aqueous diluent(s) [e.g. propylene glycol, polyethylene glycol, olive oil, ethanol, POLYSORBATE 80 (trade mark)]

Injectable compositions may comprise additional materials other than inert diluents e.g. preserving agents, wetting agents, emulsifying agents, dispersing agents, stabilizing agents (e.g. lactose), agents to assist dissolution (e.g. glutamic acid, aspartic acid).

They may be sterilized, for example, by filtration through a bacteria-retaining filter, by incorporation of sterilizing agents in the compositions or by irradiation. They may also be manufactured, for example, by freeze drying, in the form of sterile solid compositions which can be dissolved in sterile water or some other sterile diluent for injection immediately before use.

Other compositions for parenteral administration include liquids for external use, and endermic liniments (e.g. ointments), suppositories and pessaries which comprise one or more of the active compound(s) and may be prepared by known methods.

Compositions for dermal administration, especially for the treatment and prevention of alopecia and acne, include liquids for external use such as lotions, tonics, sprays, solutions, suspensions, emulsions and liniments such as ointments, gels and creams.

Such compositions may comprise one or more active ingredient(s) and at least one inert diluent(s), for example, distilled water, a lower alcohol such as ethanol, a higher alcohol such as cetanol, a poly alcohol

such as polyethylene glycol, propylene glycol, a cellulose derivative such as hydroxypropyl cellulose, animal or plant fats, vaseline, wax, silicone, a vegetable oil such as olive oil, a surfactant, or zinc oxide.

Besides inert diluents, such compositions may also comprise adjuvants (e.g. wetting agents, suspending agents, perfuming agents, preserving agents).

The following reference examples and examples illustrate the present invention.

Unless otherwise specified, "IR" spectra were measured by the KBr tablet method and "NMR" spectra were measured in a solution of $CDCl_3$.

Reference Example 1

Synthesis of 4-(4-isobutylbenzyloxy)-2,3-dimethylbenzaldehyde

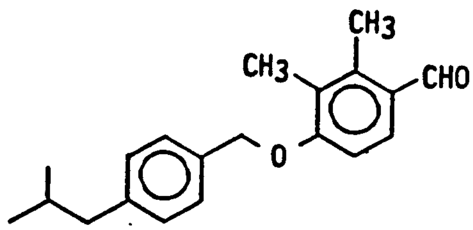

A mixture of 4-hydroxy-2,3-dimethylbenzaldehyde (220 mg), 4-isobutylbenzyl bromide (341 mg), potassium carbonate (1.38 g) and ethyl methyl ketone (10 ml) was refluxed for 6 hrs. After cooling, the reaction mixture was diluted with ethyl acetate, the solution was washed with dil.hydrochloric acid, water, successively, dried and evaporated. The residue was purified by column chromatography on silica gel (hexane:Et0Ac = 10:1) to give the title compound (383 mg) having the following physical data:

TLC: Rf 0.48 (hexane:Et0Ac = 5:1);

NMR: $\delta$ 7.64 (1H,d), 7.32 (1H,d), 7.16 (1H,d), 5.12 (2H,s), 2.60 (3H,s), 2.48 (2H,d), 2.24 (3H,s), 1.94-1.80 (1H,m), 0.90 (6H,d).

Reference example 2

Synthesis of 4-(4-isobutylbenzyloxy)-2,3-dimethylbenzoic acid

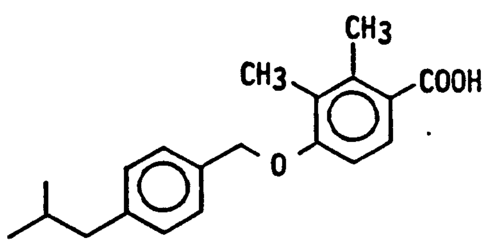

A solution of the aldehyde prepared in reference example 1 (380 mg) in acetone (5 mℓ) was cooled with ice. To the solution, Jones' reagent (2.67N; 2 mℓ) was dropped and allowed to stand. The solution was stirred for 1.5 hrs at room temperature. The reaction was stopped by adding of isopropyl alcohol. The crystals deposited were washed with hexane, dried and purified by column chromatography on silica gel (hexane-Et0Ac) to give the title compound (328 mg) having the following physical data:

TLC: Rf 0.36 (hexane:Et0Ac = 2:1);

NMR: $\delta$ 7.80 (1H,d), 7.33 (1H,d), 7.15 (1H,d), 6.90 (1H,d), 5.09 (2H,s), 2.58 (3H,s), 2.48 (2H,d), 2.26 (3H,s), 0.91 (6H,d).

Example 1

Synthesis of 4-[2-[4-(4-isobutylbenzyloxy)-2,3-dimethylbenzoylamino] phenoxy]butanoic acid ethyl ester

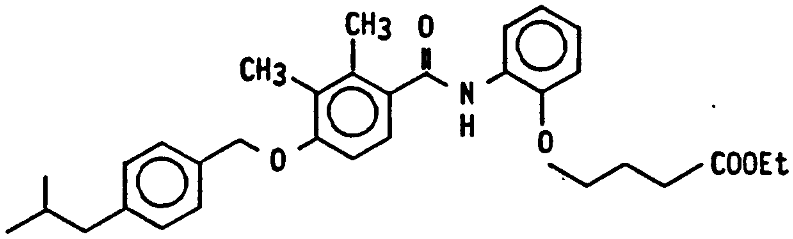

Oxalyl chloride (2 mℓ) was dropped to a solution of the carboxylic acid prepared in reference example 2 (325 mg) in methylene chloride (2 mℓ). The solution was stirred for 1 hr and evaporated. To an ice-cooled mixture of ethyl 4-(2-aminophenoxy)butanoate (232 mg), pyridine (1 mℓ) and methylene chloride (15 mℓ), the above solution was dropped. The mixture was stirred for 30 mins at the same temperature and for 1 hr at room temperature. The reaction solution was washed with water, dried and evaporated. The residue was purified by column chromatography on silica gel (hexane:Et0Ac = 5:1) to give the title compound (383 mg) having the following physical data:

TLC: Rf 0.5 (hexane:Et0Ac = 3:1);

NMR: δ 8.58-8.48 (1H,m), 8.05 (1H,s), 7.34 (2H,d), 7.16 (1H,d), 7.08-6.96 (2H,m), 6.90-6.80 (2H,m), 5.07 (2H,s), 4.14-3.96 (4H,m), 2.49 (2H,d), 2.44 (3H,s), 1.18 (3H,t), 0.91 (6H,d).

Example 2

Synthesis of 4-[2-[4-(4-isobutylbenzyloxy)-2,3-dimethylbenzoylamino] phenoxy]butanoic acid

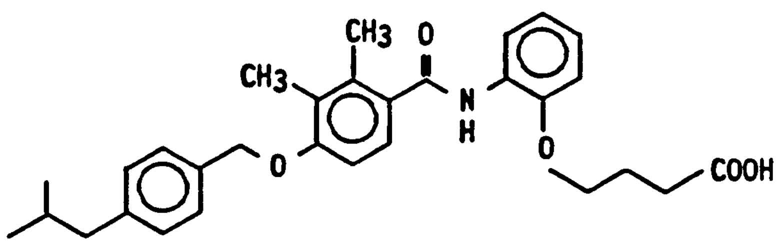

1N aq. solution of lithium hydroxide (3 mℓ) was added to a solution of the ester prepared in example 1 (380 mg) in dimethoxyethane (8 mℓ). The mixture was stirred for 30 mins at 50°C. After reaction, the solution was neutralized with dil. hydrochloric acid and was extracted with ethyl acetate. The extract was dried and evaporated. The residue obtained was recrystalized from hexane to give the title compound (317 mg) having the following physical data:

TLC: Rf 0.26 (hexane:Et0Ac = 1:1);

mp : 143°C.

Example 2(a) - 2(dd)

By the similar procedure as reference example 1, 2 and example 1 and 2, compounds having the following physical data shown in the Table II, III and IV were given.

Table II

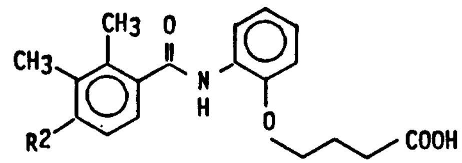

| No. | R2- | Name | TLC (Rf) | mp or MS |
|---|---|---|---|---|
| 2 (a) | 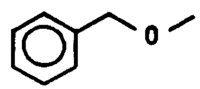 | 4-[2-(4-benzyloxy-2,3-dimethylbenzoylamino)phenoxy]butanoic acid | 0.44 ($CH_2Cl_2$:EtOAc = 7:3) | 165 - 167°C |
| 2 (b) | 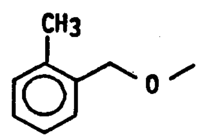 | 4-[2-[4-(2-methylbenzyloxy)-2,3-dimethylbenzoylamino]phenoxy]butanoic acid | 0.16 (hexane:EtOAc = 2:1) | 125 - 127°C |
| 2 (c) | 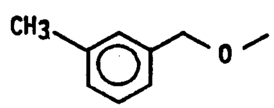 | 4-[2-[4-(3-methylbenzyloxy)-2,3-dimethylbenzoylamino]phenoxy]butanoic acid | 0.16 (hexane:EtOAc = 2:1) | 119 - 122°C |

Table II. (continued)

| No. | R2- | Name | TLC (Rf) | mp or MS |
|---|---|---|---|---|
| 2 (d) | 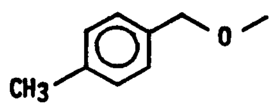 | 4-[2-[4-(4-methylbenzyloxy)-2,3-dimethylbenzoylamino]phenoxy]butanoic acid | 0.15 (hexane:EtOAc = 2:1) | 142 - 145°C |
| 2 (e) | 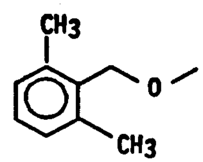 | 4-[2-[4-(2,6-dimethylbenzyloxy)-2,3-dimethylbenzoylamino]phenoxy]butanoic acid | 0.20 (hexane:EtOAc = 2:1) | 127 - 131°C |
| 2 (f) | 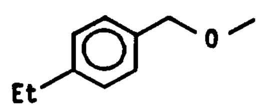 | 4-[2-[4-(4-ethylbenzyloxy)-2,3-dimethylbenzoylamino]phenoxy]butanoic acid | 0.13 (hexane:EtOAc = 2:1) | 155 - 158°C |
| 2 (g) | 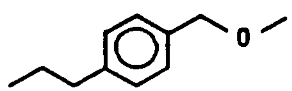 | 4-[2-[4-(4-propylbenzyloxy)-2,3-dimethylbenzoylamino]phenoxy]butanoic acid | 0.14 (hexane:EtOAc = 2:1) | 137 - 141°C |

Table II (continued)

| Example No. | R2- | Name | TLC (Rf) | mp or MS |
|---|---|---|---|---|
| 2 (h) | 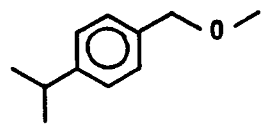 | 4-[2-[4-(4-isopropylbenzyloxy)-2,3-dimethylbenzoylamino]phenoxy]butanoic acid | 0.13 (hexane:EtOAc = 2:1) | 137 - 141°C |
| 2 (i) | 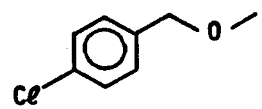 | 4-[2-[4-(4-chlorobenzyloxy)-2,3-dimethylbenzoylamino]phenoxy]butanoic acid | 0.47 ($CH_2Cl_2$:EtOAc = 7:3) | 156 - 160°C |
| 2 (j) | 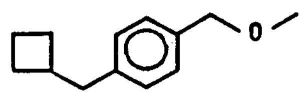 | 4-[2-[4-(4-cyclobutylmethylbenzyloxy)-2,3-dimethylbenzoylamino]phenoxy]butanoic acid | 0.17 (hexane:EtOAc = 2:1) | 146 - 147°C |
| 2 (k) | 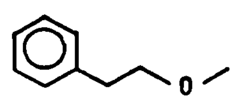 | 4-[2-[4-(2-phenylethoxy)-2,3-dimethylbenzoylamino]phenoxy]butanoic acid | 0.37 ($CHCl_3$:$CH_3OH$ = 95:5) | 141 - 142°C |

Table II (continued)

| No. | R2- | Name | TLC (Rf) | mp or MS |
|---|---|---|---|---|
| 2 (l) | 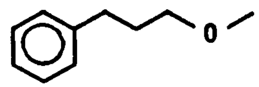 | 4-[2-[4-(3-phenylpropoxy)-2,3-dimethylbenzoylamino]phenoxy]butanoic acid | 0.35 ($CHCl_3$:$CH_3OH$ = 95:5) | 152°C |
| 2 (m) | 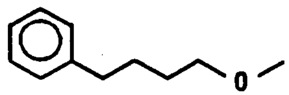 | 4-[2-[4-(4-phenylbutoxy)-2,3-dimethylbenzoylamino]phenoxy]butanoic acid | 0.37 ($CHCl_3$:$CH_3OH$ = 95:5) | 113°C |
| 2 (n) | 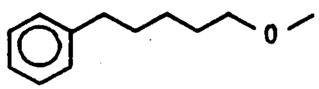 | 4-[2-[4-(5-phenylpentyloxy)-2,3-dimethylbenzoylamino]phenoxy]butanoic acid | 0.11 (hexane:EtOAc = 2:1) | MS m/z 295, 189 |
| 2 (o) | 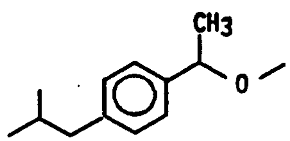 | 4-[2-[4-[1-(4-isobutylphenyl)ethoxy]-2,3-dimethylbenzoylamino]phenoxy] butanoic acid | 0.37 (hexane:EtOAc = 1:1) | MS m/z 503, 345 |

Table II (continued)

| No. | R2- | Name | TLC (Rf) | mp or MS |
|---|---|---|---|---|
| 2 (p) | 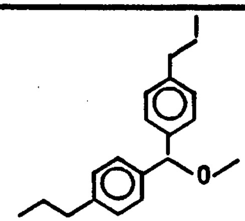 | 4-[2-[4-[bis(4-propylphenyl)methoxy]-2,3-dimethylbenzoylamino]phenoxy]butanoic acid | 0.32 (hexane:EtOAc = 1:1) | MS m/z 593, 551, 523 |
| 2 (q) | 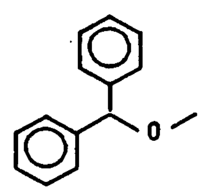 | 4-[2-(4-diphenylmethoxy)-2,3-dimethylbenzoylamino]phenoxy]butanoic acid | 0.37 (hexane:EtOAc = 1:1) | MS m/z 509, 361, 343, 315 |

Table II (continued)

| No. | R2- | Name | TLC (Rf) | mp or MS |
|---|---|---|---|---|
| 2 (r) | 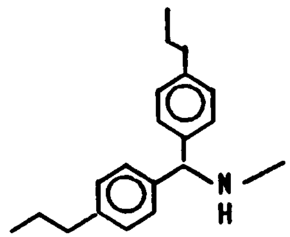 | 4-[2-[4-[bis(4-propylphenyl)methyl amino]-2,3-dimethylbenzoylamino] phenoxy]butanoic acid | 0.20 (hexane:EtOAc = 1:1) | MS m/z 592, 506, 398, 342, 251 |
| 2 (s) | 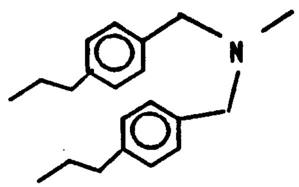 | 4-[2-[4-[N,N-bis(4-propylphenyl methyl)amino]2,3-dimethylbenzoyl amino]phenoxy]butanoic acid | Rf 0.37 (hexane:EtOAc = 1:1) | MS m/z 606, 520, 473, 413, 278, 133 |
| 2 (t) | 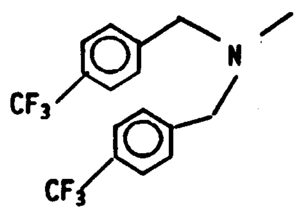 | 4-[2-[4-[N,N-bis(4-trifluoromethyl phenylmethyl)amino]-2,3-dimethyl benzoylamino]phenoxy]butanoic acid | Rf 0.28 (hexane:EtOAc = 1:1) | MS m/z 658, 455, 304, 277 159, 132 |

Table II (continued)

| No. | $R^2$- | Name | TLC (Rf) | mp or MS |
|---|---|---|---|---|
| 2 (u) | 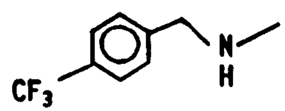 | 4-[2-[4-[N-(4-trifluoromethyl phenylmethyl)amino]-2,3-dimethylbenzoylamino]phenoxy] butanoic acid | Rf 0.20 (EtOAc) | MS m/z 500, 307 277, 263, 183, 167, 159, 118 |
| 2 (v) | 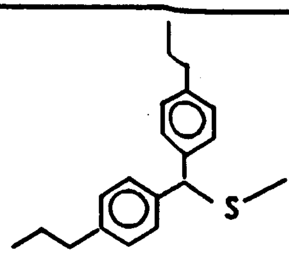 | 4-[2-[4-[bis(4-propylphenyl) methylthio]-2,3-dimethylbenzoyl amino]phenoxy]butanoic acid | Rf 0.28 (EtOAc) | MS m/z 609, 359, 313, 252, 222, 193, 167 |
| 2 (w) | 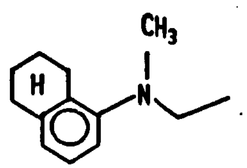 | 4-[2-[4-[N-Methyl-N-(5,6,7,8-tetrahydronaphth-1-yl)aminomethyl]-benzoylamino]phenoxy]butanoic acid | Rf 0.30 (EtOAc) | MS m/z 500, 340, 312, 306, 161 |

Table III

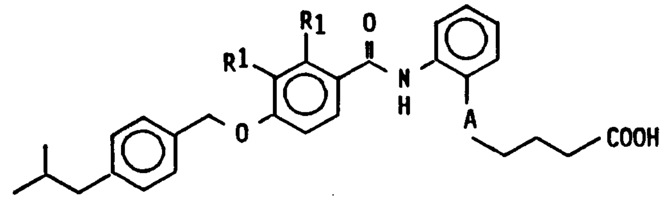

| No. | , A | Name | TLC (Rf) | mp |
|---|---|---|---|---|
| | 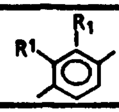 , A | | | |
| 2 (x) | 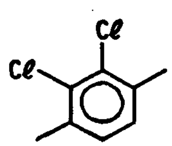 A=O | 4-[2-[4-(4-isobutylbenzyloxy)-2,3-dichlorobenzoylamino]phenoxy]butanoic acid | 0.47 (hexane:EtOAc = 1:3) | 139 - 140°C |
| 2 (y) | 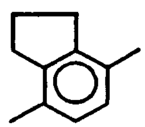 A=O | 4-[2-(7-(4-isobutylbenzyloxy)-indane-4-carbonylamino]phenoxy] -butanoic acid | 0.27 (hexane:EtOAc = 1:1) | 156 - 157°C |
| 2 (z) | 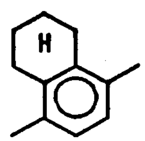 A=O | 4-[2-[4-(4-isobutylbenzyloxy)-5,6,7,8-tetrahydronaphthalene-1-carbonylamino]phenoxy]butanoic acid | 0.50 (hexane:EtOAc = 1:1) | 164°C |

Table III (continued)

| No. | 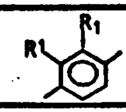 , A | | Name | TLC (Rf) | mp |
|---|---|---|---|---|---|
| 2 (aa) | 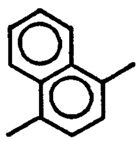 | A=O | 4-[2-[4-[4-isobutylbenzyloxy) naphthalene-1-carbonylamino]phenoxy] butanoic acid | 0.27 (hexane:EtOAc = 1:1) | 156 - 157°C |
| 2 (bb) | 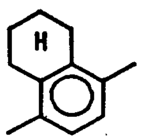 | A=S | 4-[2-[4-(4-isobutylbenzyloxy)-5,6,7,8-tetrahydronaphthalene-1-carbonylamino]phenylthio]butanoic acid | 0.39 (hexane:EtOAc = 1:1) | 135°C |

Table IV

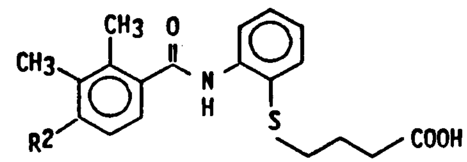

| No. | R2- | Name | TLC (Rf) | MS |
|---|---|---|---|---|
| 2 (cc) | 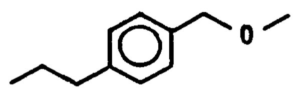 | 4-[2-[4-(4-propylbenzyloxy)-2,3-dimethylbenzoylamino)phenylthio] butanoic acid | 0.40 (hexane:EtOAc = 2:1) | m/z 491, 372, 434, 281 |
| 2 (dd) | 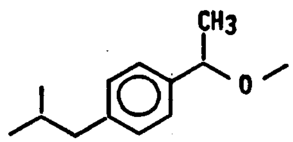 | 4-[2-[4-[1-(4-isobutylphenyl) ethoxy]-2,3-dimethylbenzoylamino] phenylthio]butanoic acid | 0.31 (hexane:EtOAc = 2:1) | m/z 519, 359, 309 |

Example 3

Synthesis of 4-[2-[4-(4-propylbenzyloxy)-2,3-dimethylbenzoylamino] phenylsulfinyl]butanoic acid

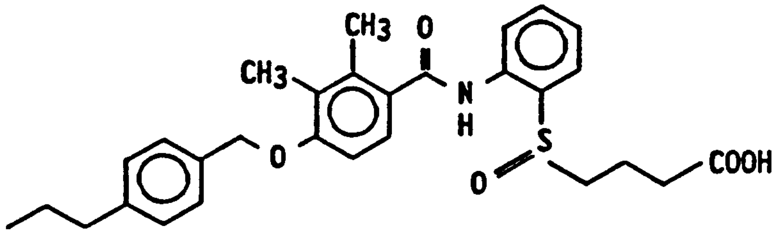

A solution of sodium periodate (3.85 g) in water (84 mℓ) was added to a solution of the compound prepared in example 2(cc) (6.3g) in methanol (340 mℓ). The mixture was stirred for 12 hrs at room temperature. To the mixture, a solution of sodium periodate (1.28 g) in water (30 mℓ) was added and the mixture was stirred for 3 hrs at room temperature. The reaction solution was filtered, and the filtrate was concentrated to dryness. Toluene was added to the residue, the mixture was distilled azeotropically. The residue was purified by column chromatography on silica gel (chloroform-methanol), and further recrystallization from a mixture of benzene and hexane to give the title compound (6.39g) having the following physical data:

TLC: Rf 0.53 (chloroform:methanol = 9:1);

IR : ν 3600-2300, 1760, 1650, 1585, 1460, 1270, 1100, 1000, 760 $cm^{-1}$.

Example 4

(-)-4-[2-(4-[1-(4-isobutylphenyl)ethoxy]-2,3-dimethylbenzoylamino]phenoxy] butanoic acid

The compounds prepared in example 2(o) (403 mg) and cinchonidine (2.36g) were dissolved into acetone (70 ml) with heating. The solution was allowed to stand to give white crystals. The crystals were gathered by filtration, and purified by recrystallization from acetone four times. The white crystals obtained were dissolved into chlorofom. The solution was washed with dil. hydrochloric acid. The oily layer was washed with water, dried and evaporated to give the title compound having the following physical data:

Appearance : white crystal ;

Optical angle of rotation : [α]D -39.6° (c = 1, $CHCl_3$).

Example 5

Sodium salt of (-)-4-[2-[4-[1-(4-isobutylphenyl)ethoxy]-2,3-dimethylbenzoylamino]phenoxy]butanoic acid

The compound prepared in example 4 was dissolved into methanol. The equivalent molar of an aq. sodium hydroxide solution was added and evaporated to give the title compound having the following data:

IR : ν 3050, 1750, 1580, 1560, 1510, 1445, 1260, 1090, 1020, 740 $cm^{-1}$

Formulation example

The following components were admixed in conventional method and punched out to obtain 100 tablets each containing 50 mg of active ingredient.

| | | |
|---|---|---|
| • 4-[2-[4-(4-isobutylbenzyloxy)-2,3-dimethylbenzoylamino]phenoxy] butanoic acid | ................ | 5 g |
| • Cellulose calcium gluconate (disintegrating agent) | ................ | 0.2 g |
| • Magnesium stearate (lubricating agent) | ................ | 0.1 g |
| • Microcrystaline cellulose | ................ | 4.7 g |

## Claims

**Claims for the following Contracting States : AT, BE, CH, DE, FR, GB, IT, LI, LU, NL, SE**

**1.** A benzoylaminophenoxybutanoic acid derivative of the general formula:

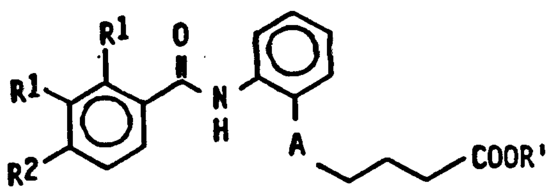

(I)

[wherein R′ represents a hydrogen atom or an alkyl group of from 1 to 4 carbon atom(s),
A represents an oxygen atom, a sulfur atom or a sulfinyl (SO) group,
the two groups $R^1$ simultaneously represent methyl groups or chlorine atoms or form, together with the carbon atoms of the benzene ring to which they are attached a cyclopentane, cyclohexane or benzene ring,
$R^2$ represents a group of the general formula:

(i)

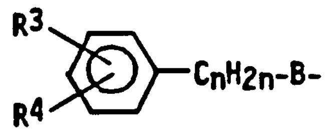

(ii)

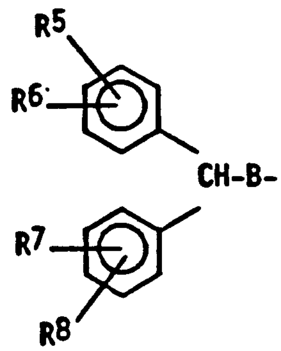

or

(iii)

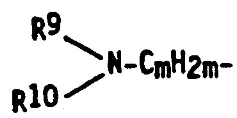

(wherein B represents an oxygen atom, a sulfur atom or a group of the general formula: $NR^{11}$ (wherein $R^{11}$ represents a hydrogen atom or an alkyl group of from 1 to 4 carbon atom(s)),
$R^3$, $R^4$, $R^5$, $R^6$, $R^7$ and $R^8$ represent, independently, a hydrogen atom, an alkyl group of from 1 to 4 carbon atom(s), a halogen atom, a trifluoromethyl group or a cyclobutylmethyl group,
m represents 0 or 1,
n represents an integer of from 1 to 5, and
$R^9$ and $R^{10}$, which may be the same or different, each represents a hydrogen atom, an alkyl group of from 1 to 5 carbon atom(s) or a group of the general formula:

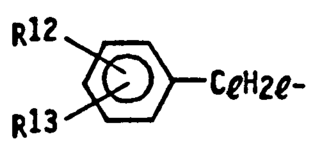

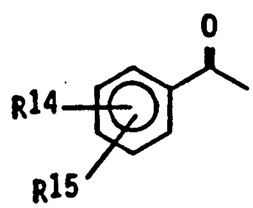

or

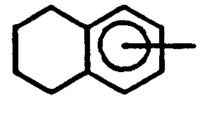

(wherein $R^{12}$, $R^{13}$, $R^{14}$ and $R^{15}$ represent, independently, a hydrogen atom, an alkyl group of from 1 to 4 carbon atom(s), a halogen atom, a trifluoromethyl group or a cyclobutylmethyl group, and
l represents an integer of from 1 to 4),
with the proviso that $R^9$ and $R^{10}$ do not simultaneously represent hydrogen atoms] or a non-toxic salt

thereof.

2. A compound according to claim 1, wherein $R^2$ is a group of the general formula (i).

3. A compound according to claim 2, wherein both groups $R^1$ are methyl groups.

4. A compound according to claim 1 or 3, which is
4-[2-(4-benzyloxy-2,3-dimethylbenzoylamino)phenoxy]butanoic acid,
4-[2-[4-(2-methylbenzyloxy)-2,3-dimethylbenzoylamino]phenoxy]butanoicacid,
4-[2-[4-(3-methylbenzyloxy)-2,3-dimethylbenzoylamino]phenoxy]butanoicacid,
4-[2-[(4-methylbenzyloxy)-2,3-dimethylbenzoylamino]phenoxy]butanoic acid,
4-[2-[4-(2,6-dimethylbenzloxy)-2,3-dimethylbenzoylamino]phenoxy]butanoic acid,
4-[2-[4-(4-ethylbenzyloxy)-2,3-dimethylbenzoylamino]phenoxy]butanoic acid,
4-[2-[4-(4-propylbenzyloxy)-2,3-dimethylbenzoylamino]phenoxy]butanoicacid,
4-[2-[4-(4-isopropylbenzyloxy)-2,3-dimethylbenzoylamino]phenoxy]butanoic acid,
4-[2-[4-(4-isobutylbenzyloxy)-2,3-dimethylbenzoylamino]phenoxy]butanoic acid,
4-[2-[4-(4-chlorobenzyloxy)-2,3-dimethylbenzoylamino]phenoxy]butanoicacid,
4-[2-[4-(4-cyclobutylmethylbenzyloxy)-2,3-dimethylbenzoylamino]phenoxy]butanoic acid
4-[2-[4-(2-phenylethoxy)-2,3-dimethylbenzoylamino]phenoxy]butanoic acid,
4-[2-[4-(3-phenylpropoxy)-2,3-dimethylbenzoylamino]phenoxy]butanoic acid,
4-[2-[4-(4-phenylbutoxy)-2,3-dimethylbenzoylamino]phenoxybutanoic acid,
4-[2-[4-(5-phenylpentyloxy)-2,3-dimethylbenzoylamino]phenoxy]butanoicacid,
4-[2-[4-[1-(4-isobutylpheny)ethoxy]-2,3-dimethylbenzoylamino]phenoxy]butanoic acid,
4-[2-[4-(4-propylbenzyloxy)-2,3-dimethylbenzoylamino)phenylthio]butanoic acid,
4-[2-[4-[1-(4-isobutylphenyl)ethoxy]-2,3-dimethylbenzoylamino]phenylthio]butanoic acid
4-[2-[4-(4-propylbenzyloxy)-2,3-dimethylbenzoylamino]phenylsulfinyl]butanoic acid or
4-[2-[4-[N-(4-trifluoromethylphenylmethyl)amino]-2,3-dimethylbenzoylamino]phenoxy]butanoic acid,
or a non-toxic salt thereof.

5. A compound according to claim 2, wherein the two groups $R^1$ represent chlorine atoms or together with the carbon atoms of the benzene ring to which they are attached, a cyclopentane, cyclohexane or benzene ring.

6. A compound according to claim 1 or 5, which is
4-[2-[4-(4-isobutylbenzyloxy)-2,3-dichlorobenzoylamino]phenoxy]butanoic acid,
4-[2-(7-(4-isobutylbenzyloxy)indane-4-carbonylamino]phenoxy]butanoic acid,
4-[2-[4-(4-isobutylbenzyloxy)-5,6,7,8-tetrahydronaphthalene-1-carbonylamino]phenoxy]butanoic acid,
4-[2-[4-[4-isobutylbenzyloxy)naphthalene-1-carbonylamino]phenoxy]butanoic acid or
4-[2-[4-(4-isobutylbenzyloxy)-5,6,7,8-tetrahydronaphthalene-1-carbonylamino]phenylthio]butanoic acid,
or a non-toxic salt thereof.

7. A compound according to claim 1, wherein $R^2$ is a group of the general formula (ii).

8. A compound according to claim 1 or 7, which is:
4-[2-[4-[bis(4-propylphenyl)methoxy]-2,3-dimethylbenzoylamino]phenoxy]butanoic acid,
4-[2-(4-diphenylmethoxy)-2,3-dimethylbenzoylamino]phenoxy]butanoic acid,
4-[2-[4-[bis(4-propylphenyl)methylamino]-2,3-dimethylbenzoylamino]phenoxy]butanoic acid or
4-[2-[4-[bis(4-propylphenyl)methylthio]-2,3-dimethylbenzoylamino]phenoxy]butanoic acid,
or a non-toxic salt thereof.

9. A compound according to claim 1, wherein $R^2$ is a group of the general formula (iii).

10. A compound according to claim 1 or 9, which is:
4-[2-[4-[N,N-bis(4-propylphenylmethyl)amino]-2,3-dimethylbenzoylamino]phenoxy]butanoic acid,
4-[2-[4-[N,N-bis(4-trifluoromethylphenylmethyl)amino]-2,3-dimethylbenzoylamino]phenoxy]butanoic acid or
4-[2-[4-[N-methyl-N-(5,6,7,8-tetrahydronaphth-1-yl]aminomethyl]-2,3-dimethylbenzoylamino]phenoxy]-butanoic acid,

or a non-toxic salt thereof.

**11.** A process for the preparation of a benzoylaminophenoxybutanoic acid derivative according to claim 1 which comprises:

(A) for the preparation of compounds of the general formula:

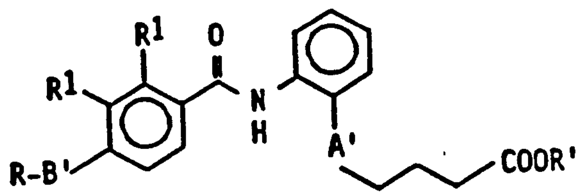

(Ia)

[wherein

A′ represents an oxygen atom or a sulfur atom,

R represents a group of the general formula:

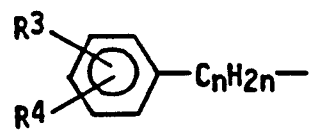

or

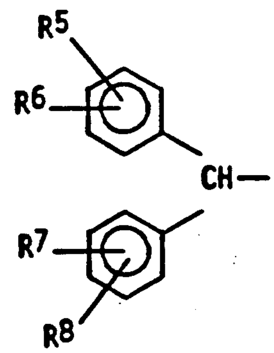

(wherein the various symbols are as defined in claim 1),

B′ represents an oxygen atom or a sulfur atom, and the other symbols are as defined in claim 1)

reacting a carboxylic acid of the general formula:

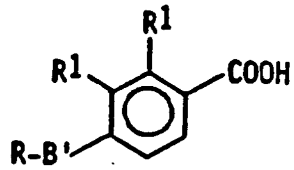

(II)

(wherein B′ is as hereinbefore defined and R and $R^1$ are as defined in claim 1) or an activated derivative thereof with an amine of general formula:

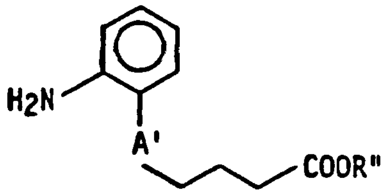

(III)

(wherein R″ represents an alkyl group of from 1 to 4 carbon atom(s) and A′ is as hereinbefore defined);
(B) for the preparation of compounds of the general formula:

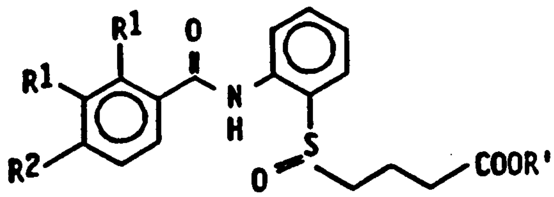

(Ib)

(wherein R′, $R^1$ and $R^2$ are as defined in claim 1) oxidizing a compound of the general formula:

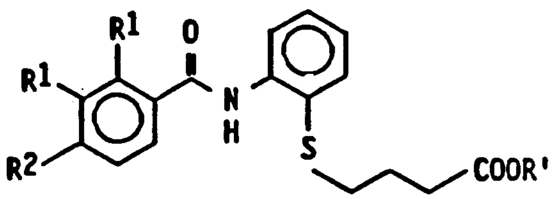

(Ib')

(wherein R′, $R^1$ and $R^2$ are as defined in claim 1) to convert the group -S- to a group -S(=O)-;
(C) for the preparation of compounds of the general formula:

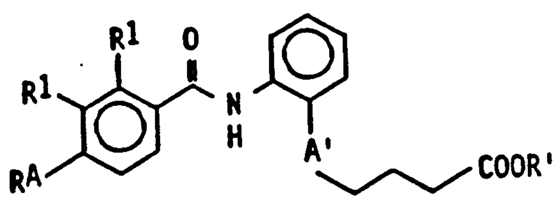

(Ic)

(wherein $R^A$ represents a group of general formula:

(i')

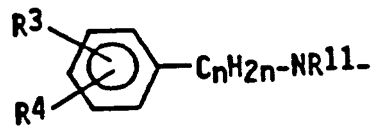

(ii')

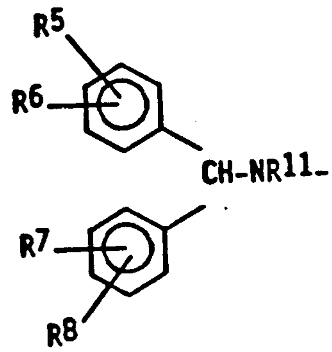

or

(iii')

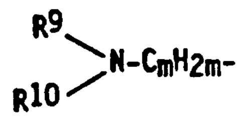

(wherein all of the symbols are as defined in claim 1), $R^1$ and $R'$ are as defined in claim 1 and $A'$ is as hereinbefore defined) reacting a compound of the general formula:

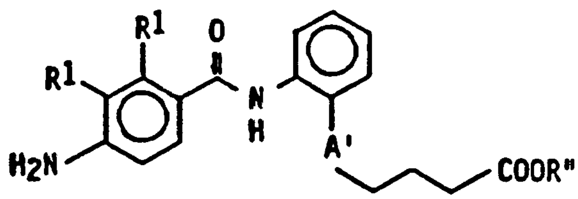

(IV)

(wherein $R^1$ is as defined in claim 1 and $A'$ and $R''$ are as hereinbefore defined) with a compound of the general formula:

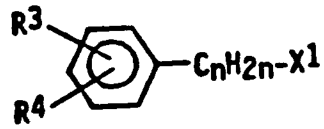

(V)

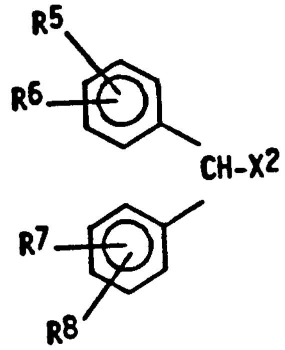

(VI)

$R^9$ - $X^3$ (VII)
$R^{10}$ - $X^4$ (VIII)

and/or

$R^{11}$ - $X^5$ (IX)

(wherein $X^1$, $X^2$, $X^3$, $X^4$ and $X^5$ each represent a halogen atom, a tosyl group or a mesyl group, and the other symbols are as defined in claim 1) or by reacting a compound of the general formula:

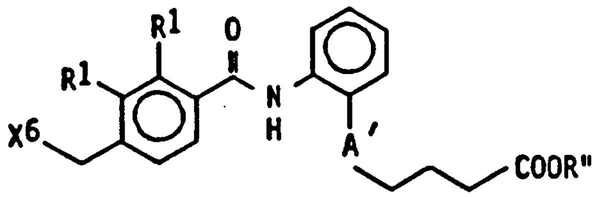

(X)

(wherein $X^5$ represents a halogen atom, a tosyl group or a mesyl group, $R^1$ is as defined in claim 1 and A' and R" are as hereinbefore defined) with a compound of general formula:

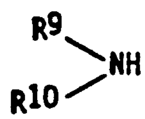

(XI)

(wherein $R^9$ and $R^{10}$ are as defined in claim 1);

and then, if desired, hydrolysing a compound of general formula (I) thus obtained to convert a group -COOR' in which R' represents an alkyl group of from 1 to 4 carbon atom(s) to a group COOH; and/or converting a compound of general formula (I) in which R' represents a hydrogen atom into a non-toxic salt thereof.

**12.** A pharmaceutical composition which comprises a benzoylaminophenoxybutanoic acid derivative according to claim 1 or a non-toxic salt thereof in association with a pharmaceutically acceptable carrier or coating.

**13.** A benzoylaminophenoxybutanoic acid derivative according to claim 1, or a non-toxic salt thereof, for

use as a medicament.

**14.** Use of a benzoylaminophenoxybutanoic acid derivative according to claim 1, or a non-toxic salt thereof, in the manufacture of a medicament for the treatment of alopecia, acne or prostatic hypertrophy.

**15.** A cosmetic composition which comprises a benzoylaminophenoxybutanoic acid derivative of the general formula (I) or a non-toxic salt thereof.

**16.** Use as a cosmetic product in the treatment of acne or alopecia of a benzoylaminophenoxybutanoic acid derivative of the general formula (I) or a non-toxic salt thereof.

**Claims for the following Contracting States : ES, GR**

**1.** A process for the preparation of a benzoylaminophenoxybutanoic acid derivative of the general formula:

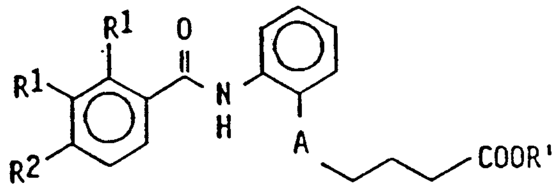

(I)

[wherein R' represents a hydrogen atom or an alkyl group of from 1 to 4 carbon atom(s),
A represents an oxygen atom, a sulfur atom or a sulfinyl (SO) group,
the two groups $R^1$ simultaneously represent methyl groups or chlorine atoms or form, together with the carbon atoms of the benzene ring to which they are attached a cyclopentane, cyclohexane or benzene ring,
$R^2$ represents a group of the general formula:

(i)

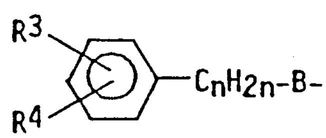

(ii)

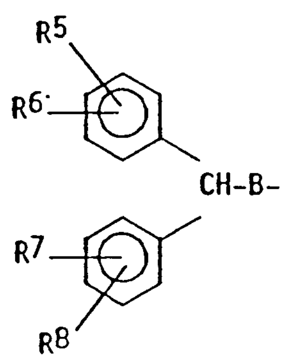

or
(iii)

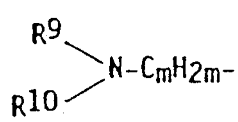

(wherein B represents an oxygen atom, a sulfur atom or a group of the general formula:
$NR^{11}$ (wherein $R^{11}$ represents a hydrogen atom or an alkyl group of from 1 to 4 carbon atom(s)),

$R^3$, $R^4$, $R^5$, $R^6$, $R^7$ and $R^8$ represent, independently, a hydrogen atom, an alkyl group of from 1 to 4 carbon atom(s), a halogen atom, a trifluoromethyl group or a cyclobutylmethyl group,
m represents 0 or 1,
n represents an integer of from 1 to 5, and
$R^9$ and $R^{10}$ , which may be the same or different, each represents a hydrogen atom, an alkyl group of from 1 to 5 carbon atom(s) or a group of the general formula:

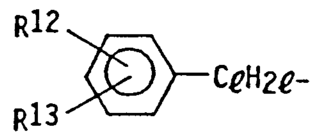

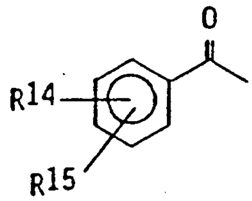

or

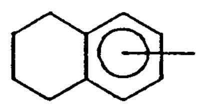

(wherein $R^{12}$, $R^{13}$, $R^{14}$ and $R^{15}$ represent, independently, a hydrogen atom, an alkyl group of from 1 to 4 carbon atom(s), a halogen atom, a trifluoromethyl group or a cyclobutylmethyl group, and
l represents an integer of from 1 to 4),
with the proviso that $R^9$ and $R^{10}$ do not simultaneously represent hydrogen atoms] or a non-toxic salt thereof;
which process comprises:
(A) for the preparation of compounds of the general formula:

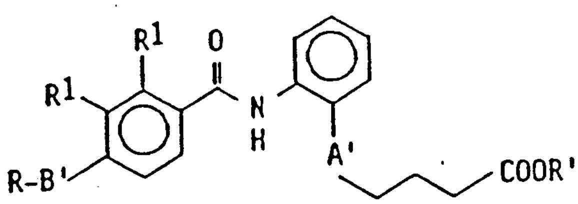

(Ia)

[wherein
A' represents an oxygen atom or a sulfur atom,
R represents a group of the general formula:

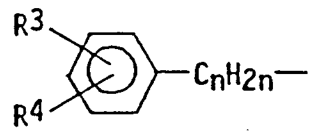

or

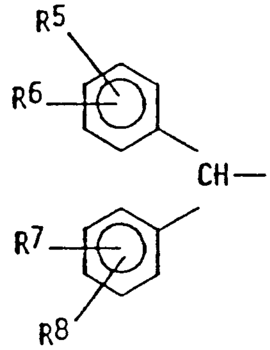

(wherein the various symbols are as hereinbefore defined), B' represents an oxygen atom or a sulfur atom, and the other symbols are as hereinbefore defined) reacting a carboxylic acid of the general formula:

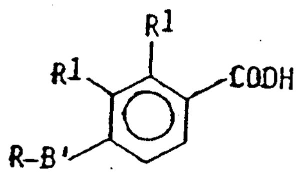

(II)

(wherein the various symbols are as hereinbefore defined) or an activated derivative thereof with an amine of general formula:

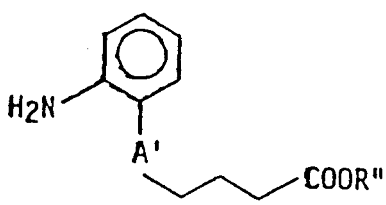

(III)

(wherein R" represents an alkyl group of from 1 to 4 carbon atom(s) and A' is as hereinbefore defined);
(B) for the preparation of compounds of the general formula:

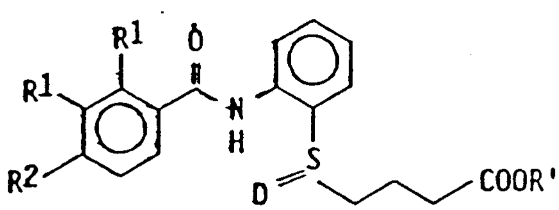

(Ib)

(wherein R', $R^1$ and $R^2$ are as hereinbefore defined) oxidizing a compound of the general formula:

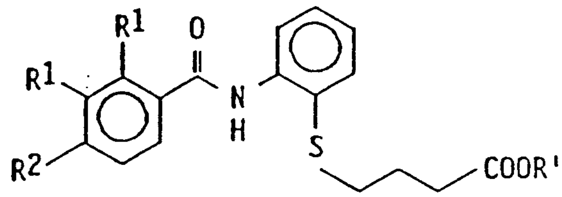

(Ib')

(wherein R', $R^1$ and $R^2$ are as hereinbefore defined) to convert the group -S- to a group -S(=O)-;
(C) for the preparation of compounds of the general formula:

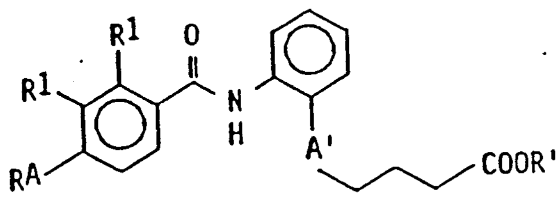

(Ic)

(wherein $R^A$ represents a group of general formula:

(i')

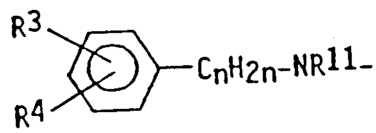

(ii')

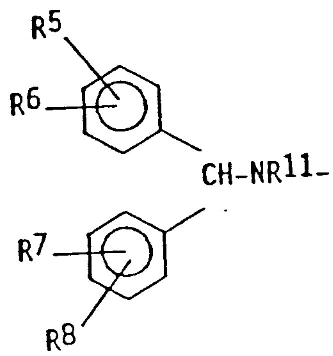

or
(iii')

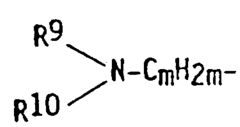

(wherein all of the symbols are as hereinbefore defined), $R^1$, R' and A' are as hereinbefore defined) reacting a compound of the general formula:

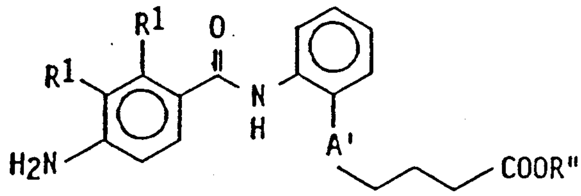

(IV)

(wherein $R^1$, A' and R'' are as hereinbefore defined) with a compound of the general formula:

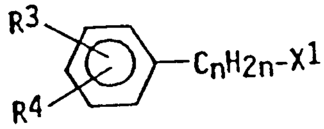

(V)

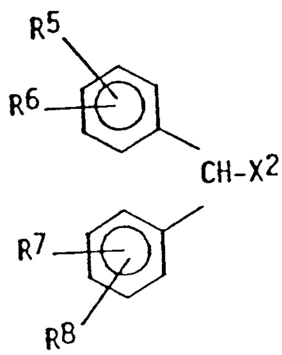

(VI)

$R^9 - X^3$ (VII)
$R^{10} - X^4$ (VIII)

and/or

$R^{11} - X^5$ (IX)

(wherein $X^1$, $X^2$, $X^3$, $X^4$ and $X^5$ each represent a halogen atom, a tosyl group or a mesyl group, and the other symbols are as hereinbefore defined) or by reacting a compound of the general formula:

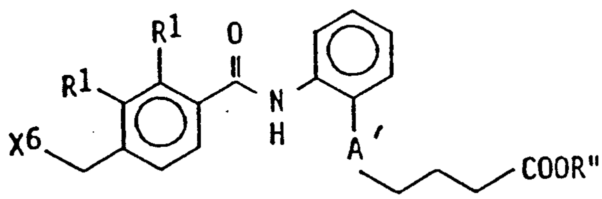

(X)

(wherein $X^5$ represents a halogen atom, a tosyl group or a mesyl group, $R^1$, A' and R'' are as hereinbefore defined) with a compound of general formula:

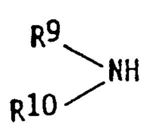

(XI)

(wherein $R^9$ and $R^{10}$ are as hereinbefore defined);

and then, if desired, hydrolysing a compound of general formula (I) thus obtained to convert a group -COOR' in which R' represents an alkyl group of from 1 to 4 carbon atom(s) to a group COOH; and/or converting a compound of general formula (I) in which R' represents a hydrogen atom into a non-toxic salt thereof.

**2.** A process according to claim 1, for the preparation of a compound of formula (I) wherein $R^2$ is a group of the general formula (i), or a non-toxic salt thereof.

**3.** A process according to claim 2, for the preparation of a compound of formula (I) wherein both groups $R^1$ are methyl groups, or a non-toxic salt thereof.

**4.** A process according to claim 1 or 3, for the preparation of a compound of formula (I) which is
4-[2-(4-benzyloxy-2,3-dimethylbenzoylamino)phenoxy]butanoic acid,
4-[2-[4-(2-methylbenzyloxy)-2,3-dimethylbenzoylamino]phenoxy]butanoicacid,
4-[2-[4-(3-methylbenzyloxy)-2,3-dimethylbenzoylamino]phenoxy]butanoicacid,
4-[2-[(4-methylbenzyloxy)-2,3-dimethylbenzoylamino]phenoxy]butanoic acid,
4-[2-[4-(2,6-dimethylbenzloxy)-2,3-dimethylbenzoylamino]phenoxy]butanoic acid,
4-[2-[4-(4-ethylbenzyloxy)-2,3-dimethylbenzoylamino]phenoxy]butanoic acid,
4-[2-[4-(4-propylbenzyloxy)-2,3-dimethylbenzoylamino]phenoxy]butanoicacid,
4-[2-[4-(4-isopropylbenzyloxy)-2,3-dimethylbenzoylamino]phenoxy]butanoic acid,
4-[2-[4-(4-isobutylbenzyloxy)-2,3-dimethylbenzoylamino]phenoxy]butanoic acid,
4-[2-[4-(4-chlorobenzyloxy)-2,3-dimethylbenzoylamino]phenoxy]butanoicacid,
4-[2-[4-(4-cyclobutylmethylbenzyloxy)-2,3-dimethylbenzoylamino]phenoxy]butanoic acid
4-[2-[4-(2-phenylethoxy)-2,3-dimethylbenzoylamino]phenoxy]butanoic acid,
4-[2-[4-(3-phenylpropoxy)-2,3-dimethylbenzoylamino]phenoxy]butanoic acid,
4-[2-[4-(4-phenylbutoxy)-2,3-dimethylbenzoylamino]phenoxy]butanoic acid,
4-[2-[4-(5-phenylpentyloxy)-2,3-dimethylbenzoylamino]phenoxy]butanoicacid,
4-[2-[4-[1-(4-isobutylpheny)ethoxy]-2,3-dimethylbenzoylamino]phenoxy]butanoic acid,
4-[2-[4-(4-propylbenzyloxy)-2,3-dimethylbenzoylamino)phenylthio]butanoic acid,
4-[2-[4-[1-(4-isobutylphenyl)ethoxy]-2,3-dimethylbenzoylamino]phenylthio]butanoic acid
4-[2-[4-(4-propylbenzyloxy)-2,3-dimethylbenzoylamino]phenylsulfinyl]butanoic acid or
4-[2-[4-[N-(4-trifluoromethylphenylmethyl)amino]-2,3-dimethylbenzoylamino)phenoxy]butanoic acid,
or a non-toxic salt thereof.

**5.** A process according to claim 2, for the preparation of a compound of formula (I) wherein the two groups $R^1$ represent chlorine atoms or together with the carbon atoms of the benzene ring to which they are attached, a cyclopentane, cyclohexane or benzene ring, or a non-toxic salt thereof.

**6.** A process according to claim 1 or 5, for the preparation of a compound of formula (I) which is
4-[2-[4-(4-isobutylbenzyloxy)-2,3-dichlorobenzoylamino]phenoxy]butanoic acid,
4-[2-(7-(4-isobutylbenzyloxy)indane-4-carbonylamino]phenoxy]butanoic acid,
4-[2-[4-(4-isobutylbenzyloxy)-5,6,7,8-tetrahydronaphthalene-1-carbonylamino]phenoxy]butanoic acid,
4-[2-[4-[4-isobutylbenzyloxy)naphthalene-1-carbonylamino]phenoxy]butanoic acid or
4-[2-[4-(4-isobutylbenzyloxy)-5,6,7,8-tetrahydronaphthalene-1-carbonylamino]phenylthio]butanoic acid,
or a non-toxic salt thereof.

**7.** A process according to claim 1, for the preparation of a compound of formula (I) wherein $R^2$ is a group of the general formula (ii), or a non-toxic salt thereof.

**8.** A process according to claim 1 or 7, for the preparation of a compound of formula (I) which is:
4-[2-[4-[bis(4-propylphenyl)methoxy]-2,3-dimethylbenzoylamino]phenoxy]butanoic acid,
4-[2-(4-diphenylmethoxy)-2,3-dimethylbenzoylamino]phenoxy]butanoic acid,
4-[2-[4-[bis(4-propylphenyl)methylamino]-2,3-dimethylbenzoylamino]phenoxy]butanoic acid or
4-[2-[4-[bis(4-propylphenyl)methylthio]-2,3-dimethylbenzoylamino]phenoxy]butanoic acid,
or a non-toxic salt thereof.

**9.** A process according to claim 1, for the preparation of a compound of formula (I) wherein $R^2$ is a group

of the general formula (iii), or a non-toxic salt thereof.

**10.** A process according to claim 1 or 9, for the preparation of a compound of formula (I) which is:
4-[2-[4-[N,N-bis(4-propylphenylmethyl)amino]-2,3-dimethylbenzoylamino]phenoxy]butanoic acid,
4-[2-[4-[N,N-bis(4-trifluoromethylphenylmethyl)amino]-2,3-dimethylbenzoylamino]phenoxy]butanoic acid or
4-[2-[4-[N-methyl-N-(5,6,7,8-tetrahydronaphth-1-yl]aminomethyl]-2,3-dimethylbenzoylamino]phenoxy]-butanoic acid,
or a non-toxic salt thereof.

**11.** A cosmetic composition which comprises a benzoylaminophenoxybutanoic acid derivative of formula (I) as defined in claim 1 or a non-toxic salt thereof.

**Revendications**
**Revendications pour les Etats contractants suivants : AT, BE, CH, DE, FR, GB, IT, LI, LU, NL, SE**

**1.** Dérivé de l'acide benzoylaminophénoxybutanoïque de formule générale:

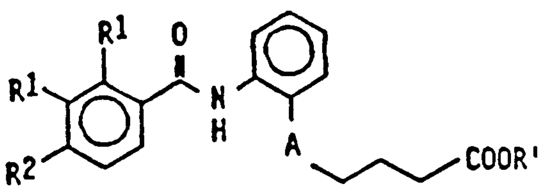

(I)

[dans laquelle R' représente un atome d'hydrogène ou un groupe alkyle de 1 à 4 atomes de carbone, A représente un atome d'oxygène, un atome de soufre ou un groupe sulfinyle (SO), les deux groupes $R^1$ représentent simultanément des groupes méthyle ou des atomes de chlore ou bien forment ensemble avec les atomes de carbone du noyau benzène auxquels ils sont attachés un noyau cyclopentane, cyclohexane ou benzène,
$R^2$ représente un groupe de formule générale :

(i)

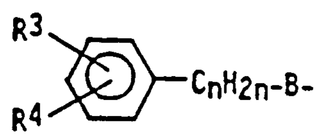

(ii)

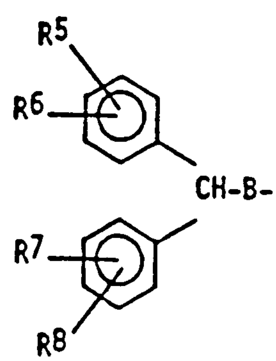

ou

(iii)

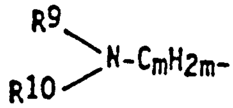

(où B représente un atome d'oxygène, un atome de soufre ou un groupe de formule générale : $NR^{11}$ (dans laquelle $R^{11}$ représente un atome d'hydrogène ou un groupe alkyle de 1 à 4 atomes de carbone),

$R^3$, $R^4$, $R^5$, $R^6$, $R^7$ et $R^8$ représentent, indépendamment, un atome d'hydrogène, un groupe alkyle de 1 à 4 atomes de carbone, un atome d'halogène, un groupe trifluorométhyle ou un groupe cyclobutylméthyle,

m représente 0 ou 1,

n représente un nombre entier de 1 à 5, et

$R^9$ et $R^{10}$ qui peuvent être identiques ou différents, représentent chacun un atome d'hydrogène, un groupe alkyle de 1 à 5 atomes de carbone ou un groupe de formule générale :

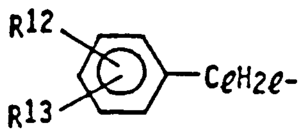

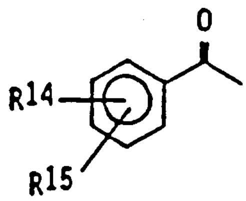

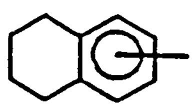

(où $R^{12}$, $R^{13}$, $R^{14}$ et $R^{15}$ représentent indépendamment un atome d'hydrogène, un groupe alkyle de 1 à 4 atomes de carbone, un atome d'halogène, un groupe trifluorométhyle ou un groupe cyclobutylméthyle, et

l représente un nombre entier de 1 à 4),

à la condition que $R^9$ et $R^{10}$ ne représentent pas simultanément des atomes d'hydrogène] ou un sel non toxique dudit dérivé.

**2.** Un composé selon la revendication 1, selon lequel $R^2$ est un groupe de formule générale (i).

**3.** Un composé selon la revendication 2, selon lequel les deux groupes $R^1$ sont des groupes méthyle.

**4.** Un composé selon la revendication 1 ou 3, qui est
l'acide 4-[2-(4-benzyloxy-2,3-diméthylbenzoylamino)phénoxy]butanoïque,
l'acide 4-[2-(4-(2-méthylbenzyloxy)-2,3-diméthylbenzoylamino]-phénoxy]butanoïque,
l'acide 4-[2-[4-(3-méthylbenzyloxy)-2,3-diméthylbenzoylamino]-phénoxy]butanoïque,
l'acide 4-[2-[(4-méthylbenzyloxy)-2,3-diméthylbenzoylamino]phénoxy] butanoïque,
l'acide 4-[2-[4-(2,6-diméthylbenzyloxy)-2,3-diméthylbenzoylamino]-phénoxy]butanoïque,
l'acide 4-[2-[4-(4-éthylbenzyloxy)-2,3-diméthylbenzoylamino]-phénoxy]butanoïque,
l'acide 4-[2-[4-(4-propylbenzyloxy)-2,3-diméthylbenzoylamino]-phénoxy]butanoïque,
l'acide 4-[2-[4-(4-isopropylbenzyloxy)-2,3-diméthylbenzoylamino]-phénoxy]butanoïque,
l'acide 4-[2-[4-(4-isobutylbenzyloxy)-2,3-diméthylbenzoylamino]-phénoxy]butanoïque,
l'acide 4-[2-[4-(4-chlorobenzyloxy)-2,3-diméthylbenzoylamino]-phénoxy]butanoïque,

l'acide 4-[2-[4-(4-cyclobutylméthylbenzyloxy)-2,3-diméthylbenzoylamino]phénoxy]butanoïque,
l'acide 4-[2-[4-(2-phényléthoxy)-2,3-diméthylbenzoylamino]phénoxy]-butanoïque,
l'acide 4-[2-[4-(3-phénylpropoxy)-2,3-diméthylbenzoylamino]phénoxy] butanoïque,
l'acide 4-[2-[4-(4-phénylbutoxy)-2,3-diméthylbenzoylamino]phénoxy] butanoïque,
l'acide 4-[2-[4-(5-phénylpentyloxy)-2,3-diméthylbenzoylamino]-phénoxy]butanoïque,
l'acide 4-[2-[4-[1-(4-isobutylphényl)éthoxy]-2,3-diméthylbenzoylamino]phénoxy]butanoïque,
l'acide 4-[2-[4-(4-propylbenzyloxy)-2,3-diméthylbenzoylamino)-phénylthio]butanoïque,
l'acide 4-[2-[4-[1-(4-isobutylphényl)éthoxy]-2,3-diméthylbenzoylamino]phénylthio]butanoïque,
l'acide 4-[2-[4-(4-propylbenzyloxy)-2,3-diméthylbenzoylamino]-phénylsulfinyl]butanoïque ou
l'acide 4-[2-[4-[N-(4-trifluorométhylphénylméthyl)amino]-2,3-diméthylbenzoylamino]phénoxy]butanoïque ou un sel non toxique de l'acide.

**5.** Un composé selon la revendication 2, selon lequel les deux groupes $R^1$ représentent des atomes de chlore ou, ensemble avec les atomes de carbone du noyau benzène auxquels ils sont attachés, un noyau cyclopentane, cyclohexane ou benzène.

**6.** Un composé selon la revendication 1 ou 5, qui est
l'acide 4-[2-[4-(4-isobutylbenzyloxy)-2,3-dichlorobenzoylamino]-phénoxy]butanoïque,
l'acide 4-[2-(7-(4-isobutylbenzyloxy)indane-4-carbonylamino]-phénoxy]butanoïque,
l'acide 4-[-2-[4-(4-isobutylbenzyloxy)-5,6,7,8-tétrahydronaphtalène-1-carbonylamino]phénoxy]-butanoïque,
l'acide 4-[2-[4-[4-isobutylbenzyloxy)naphtalène-1-carbonylamino]-phénoxy]butanoïque, ou
l'acide 4-[2-[4-(4-isobutylbenzyloxy)-5,6,7,8-tétrahydronaphtalène-1-carbonylamino]phénylthio]-butanoïque ou un sel non toxique de l'acide.

**7.** Un composé selon la revendication 1, selon lequel $R^2$ est un groupe de formule générale (ii).

**8.** Un composé selon la revendication 1 ou 7, qui est :
l'acide 4-[2-[4-[bis(4-propylphényl)méthoxy]-2,3-diméthylbenzoylamino]phénoxy]butanoïque,
l'acide 4-[2-(4-diphénylméthoxy)-2,3-diméthylbenzoylamino]phénoxy] butanoïque,
l'acide 4-[2-[4-[bis(4-propylphényl)méthylamino]-2,3-diméthylbenzoylamino]phénoxy]butanoïque ou
l'acide 4-[2-[4-[bis(4-propylphényl)méthylthio]-2,3-diméthylbenzoylamino]phénoxy]butanoïque ou un sel non toxique de l'acide.

**9.** Un composé selon la revendication 1, selon lequel $R^2$ est un groupe de formule générale (iii).

**10.** Un composé selon la revendication 1 ou 9, qui est :
l'acide 4-[2-[4-[N,N-bis(4-propylphénylméthyl)amino]-2,3-diméthylbenzoylamino]phénoxy]butanoïque,
l'acide 4-[2-[4-[N,N-bis(4-trifluorométhylphénylméthyl)amino]-2,3-diméthylbenzoylamino]phénoxy]-butanoïque ou
l'acide 4-[2-[4-[N-méthyl-N-(5,6,7,8-tétrahydronapht-1-yl]aminométhyl]-2,3-diméthylbenzoylamino]-phénoxy]butanoïque ou un sel non toxique de l'acide.

**11.** Un procédé de préparation d'un dérivé de l'acide benzoylaminophénoxybutanoïque selon la revendication 1, qui comprend :
(A) pour la préparation des composés de formule générale :

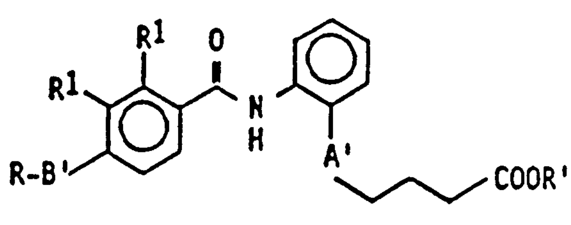

(Ia)

[dans laquelle
A' représente un atome d'oxygène ou un atome de soufre,

R représente un groupe de formule générale :

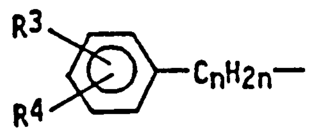

ou

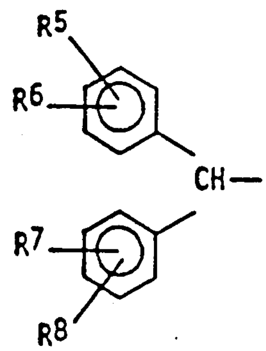

(où les divers symboles sont comme définis dans la revendication 1),
B' représente un atome d'oxygène ou un atome de soufre, et les autres symboles sont comme définis dans la revendication 1], la réaction d'un acide carboxylique de formule générale :

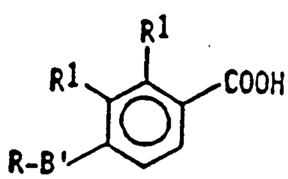

(II)

(dans laquelle B' est un comme défini précédemment et R et $R^1$ sont comme définis dans la revendication 1) ou d'un dérivé activé de cet acide avec une amine de formule générale :

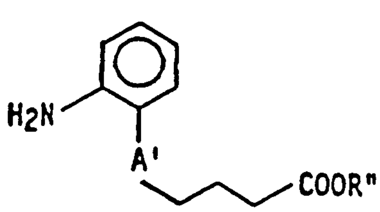

(III)

(dans laquelle R" représente un groupe alkyle de 1 à 4 atomes de carbone et A' est comme défini précédemment) ;
(B) pour la préparation des composés de formule générale :

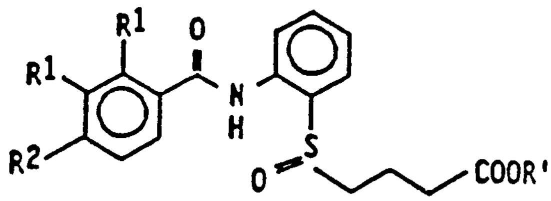

(Ib)

(dans laquelle R', $R^1$ et $R^2$ sont comme définis dans la revendication 1), l'oxydation d'un composé de formule générale :

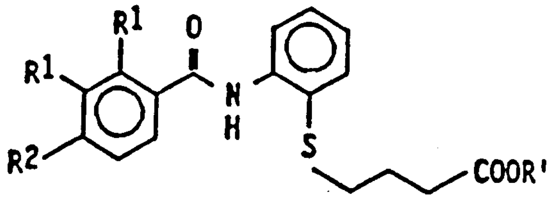

(Ib')

(dans laquelle R', $R^1$ et $R^2$ sont comme définis dans la revendication 1) pour convertir le groupe -S- en un groupe -S( = O)- ;
(C) pour la préparation des composés de formule générale :

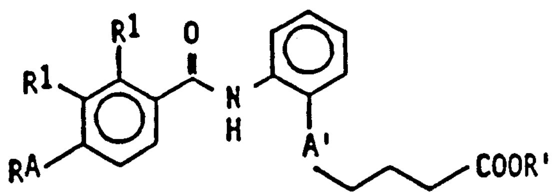

(Ic)

(dans laquelle $R^A$ représente un groupe de formule générale :

(1')

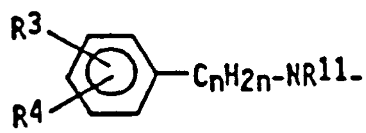

(II')

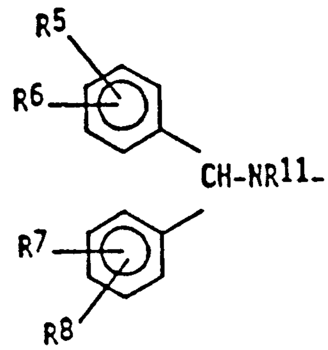

ou

(III')

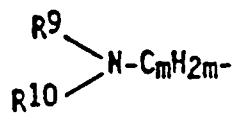

(où tous les symboles sont comme définis dans la revendication 1), $R^1$ et R' sont comme définis dans la revendication 1 et A' est comme défini précédemment), la réaction d'un composé de formule générale :

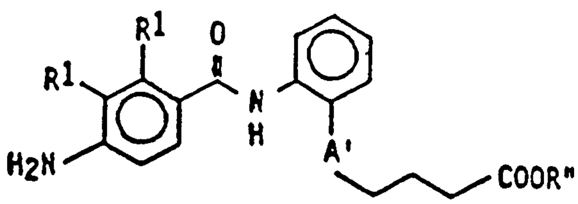

(IV)

(dans laquelle $R^1$ est comme défini dans la revendication 1 et A' et R" sont comme définis précédemment) avec un composé de formule générale :

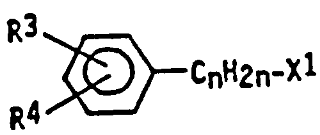

(V)

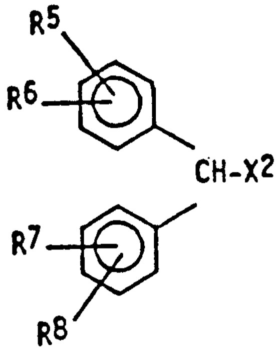

(VI)

$R^9$ - $X^3$ (VII)

$R^{10}$ - $X^4$ (VIII)

et/ou

$R^{11}$ - $X^5$ (IX)

(où $X^1$, $X^2$, $X^3$, $X^4$ et $X^5$ représentent chacun un atome d'halogène, un groupe tosyle ou un groupe mésyle, et les autres symboles sont comme définis dans la revendication 1) ou la réaction d'un composé de formule générale :

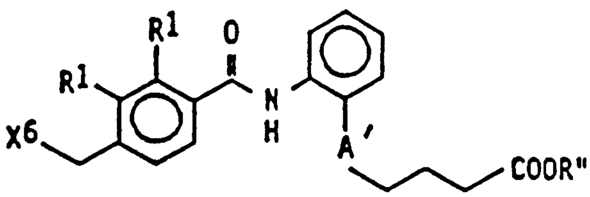

(X)

dans laquelle $X^5$ représente un atome d'halogène, un groupe tosyle ou un groupe mésyle, $R^1$ est comme défini dans la revendication 1 et A' et R" sont comme définis précédemment avec un composé de formule générale :

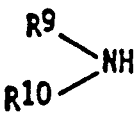

(XI)

(dans laquelle $R^9$ et $R^{10}$ sont comme définis dans la revendication 1) ;

et ensuite, si on le désire, l'hydrolyse d'un composé de formule générale (I) ainsi obtenu pour convertir le groupe -COOR' dans lequel R' représente un groupe alkyle de 1 à 4 atomes de carbone en un groupe COOH ; et/ou la conversion d'un composé de formule générale (I) dans laquelle R' représente un atome d'hydrogène en un sel non toxique correspondant.

**12.** Une composition pharmaceutique qui comprend un dérivé de l'acide benzoylaminophénoxybutanoïque selon la revendication 1 ou un sel non toxique de ce dérivé en association avec un support ou enrobage pharmaceutiquement acceptable.

**13.** Un dérivé de l'acide benzoylaminophénoxybutanoïque selon la revendication 1, ou un sel non toxique

de ce dérivé à utiliser comme médicament.

**14.** Utilisation d'un dérivé de l'acide benzoylaminophénoxybutanoïque selon la revendication 1, ou d'un sel non toxique de ce dérivé dans la fabrication d'un médicament pour le traitement de l'alopécie, de l'acné ou de l'hypertrophie prostatique.

**15.** Une composition cosmétique qui comprend un dérivé de l'acide benzoylaminophénoxybutanoïque de formule générale (I) ou un sel non toxique de ce dérivé.

**16.** Utilisation comme un produit cosmétique dans le traitement de l'acné ou de l'alopécie d'un dérivé de l'acide benzoylaminophénoxybutanoïque de formule générale (I) ou d'un sel non toxique de ce dérivé.

**Revendications pour les Etats contractants suivants : ES, GR**

**1.** Un procédé de préparation d'un dérivé de l'acide benzoylaminophénoxybutanoïque de formule générale :

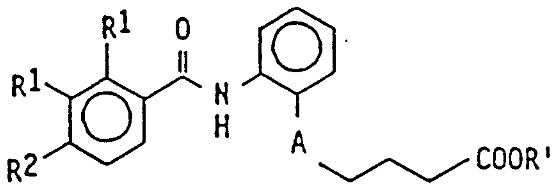

(I)

[dans laquelle R' représente un atome d'hydrogène ou un groupe alkyle de 1 à 4 atomes de carbone,
A représente un atome d'oxygène, un atome de soufre ou un groupe sulfinyle (SO),
les deux groupes $R^1$ représentent simultanément des groupes méthyle ou des atomes de chlore ou bien forment ensemble avec les atomes de carbone du noyau benzène auxquels ils sont attachés un noyau cyclopentane, cyclohexane ou benzène,
$R^2$ représente un groupe de formule générale :

(i)

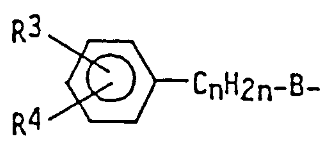

(ii)

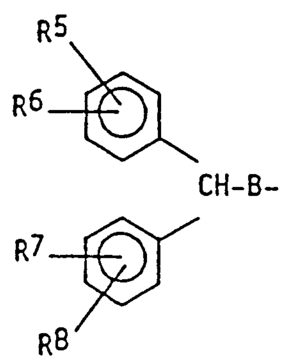

ou (iii) 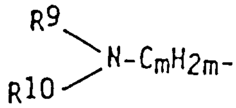

(où B représente un atome d'oxygène, un atome de soufre ou un groupe de formule générale : $NR^{11}$ (dans laquelle $R^{11}$ représente un atome d'hydrogène ou un groupe alkyle de 1 à 4 atomes de carbone),
$R^3$, $R^4$, $R^5$, $R^6$, $R^7$ et $R^8$ représentent, indépendamment, un atome d'hydrogène, un groupe alkyle de 1 à 4 atomes de carbone, un atome d'halogène, un groupe trifluorométhyle ou un groupe cyclobutylméthyle,
m représente 0 ou 1,
n représente un nombre entier de 1 à 5, et
$R^9$ et $R^{10}$ qui peuvent être identiques ou différents, représentent chacun un atome d'hydrogène, un groupe alkyle de 1 à 5 atomes de carbone ou un groupe de formule générale :

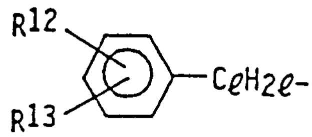

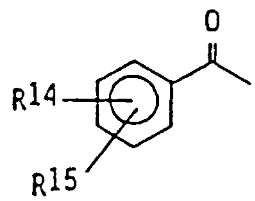

ou 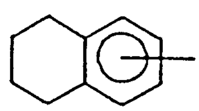

(où $R^{12}$, $R^{13}$, $R^{14}$ et $R^{15}$ représentent indépendamment un atome d'hydrogène, un groupe alkyle de 1 à 4 atomes de carbone, un atome d'halogène, un groupe trifluorométhyle ou un groupe cyclobutylméthyle, et
l représente un nombre entier de 1 à 4)),
à la condition que $R^9$ et $R^{10}$ ne représentent pas simultanément des atomes d'hydrogène] ou d'un sel non toxique dudit dérivé ; ce procédé comprenant :

(A) pour la préparation des composés de formule générale :

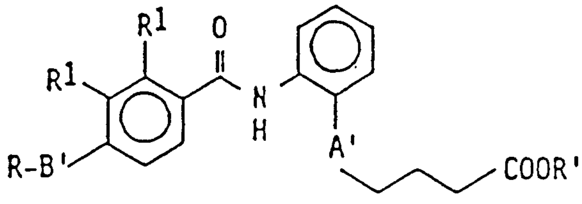 (Ia)

[dans laquelle
A' représente un atome d'oxygène ou un atome de soufre,

R représente un groupe de formule générale :

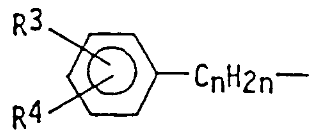

ou

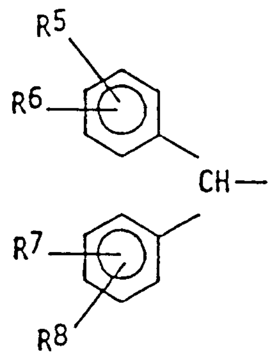

(où les divers symboles sont comme définis précédemment),
B' représente un atome d'oxygène ou un atome de soufre, et les autres symboles sont comme définis précédemment], la réaction d'un acide carboxylique de formule générale :

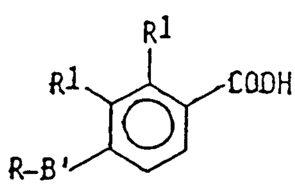

(II)

(dans laquelle les divers symboles sont comme définis précédemment) ou d'un dérivé activé de acide avec une amine de formule générale :

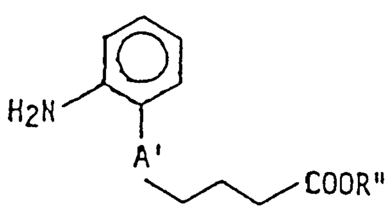

(III)

(dans laquelle R" représente un groupe alkyle de 1 à 4 atomes de carbone et A' est comme défini précédemment) ;
(B) pour la préparation des composés de formule générale :

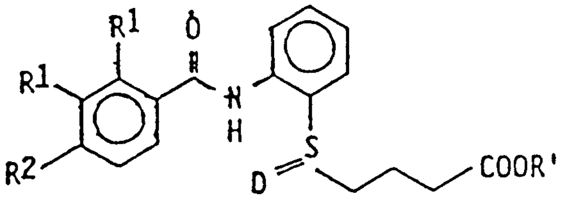

(Ib)

(dans laquelle R', $R^1$ et $R^2$ sont comme définis précédemment), l'oxydation d'un composé de formule générale :

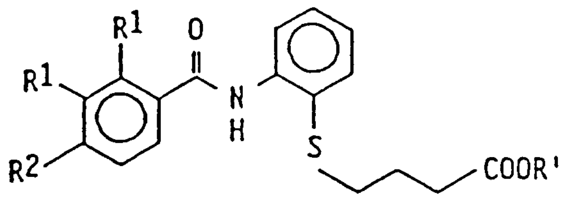

(Ib')

(dans laquelle R', $R^1$ et $R^2$ sont comme définis précédemment) pour convertir le groupe -S- en un groupe -S(=O)- ;
(C) pour la préparation des composés de formule générale :

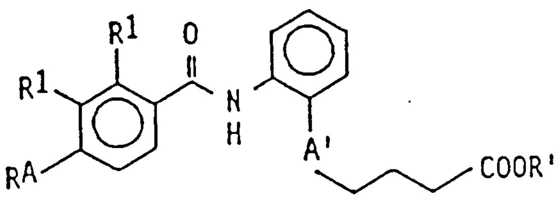

(Ic)

(dans laquelle $R^A$ représente un groupe de formule générale :

(i')

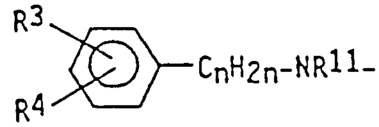

(ii')

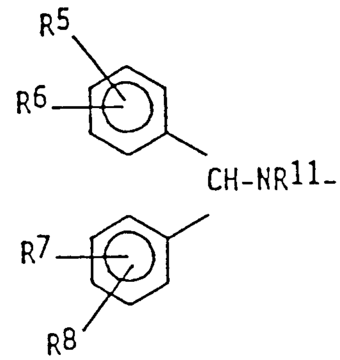

ou

(iii')

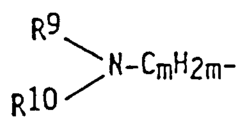

(où tous les symboles sont comme définis précédemment), $R^1$, R' et A' sont comme définis précédemment), la réaction d'un composé de formule générale :

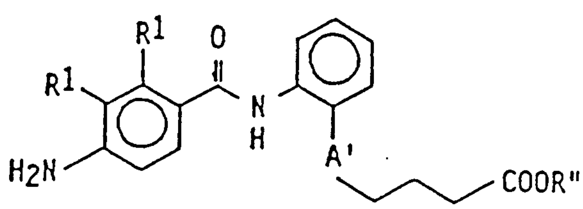

(IV)

(dans laquelle $R^1$, A' et R" sont comme définis précédemment) avec un composé de formule générale :

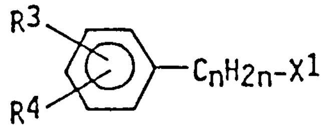

(V)

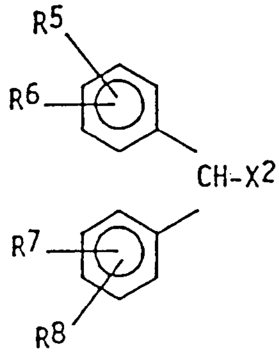

(VI)

$R^9$ - $X^3$ (VII)

$R^{10}$ - $X^4$ (VIII)

et/ou

$R^{11}$ - $X^5$ (IX)

(où $X^1$, $X^2$, $X^3$, $X^4$ et $X^5$ représentent chacun un atome d'halogène, un groupe tosyle ou un groupe mésyle, et les autres symboles sont comme définis précédemment) ou la réaction d'un composé de formule générale :

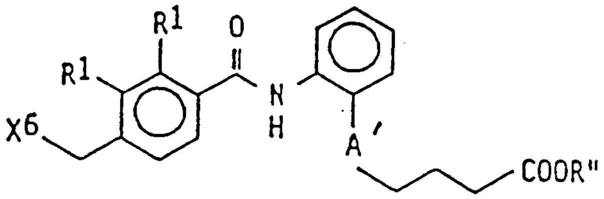

(X)

(dans laquelle $X^5$ représente un atome d'halogène, un groupe tosyle ou un groupe mésyle, $R^1$ A' et R" sont comme définis précédemment) avec un composé de formule générale :

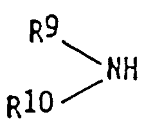

(XI)

(dans laquelle $R^9$ et $R^{10}$ sont comme définis précédemment) ;

et ensuite, si on le désire, l'hydrolyse d'un composé de formule générale (I) ainsi obtenue peut convertir le groupe -COOR' dans lequel R' représente un groupe alkyle de 1 à 4 atomes de carbone en un groupe COOH ; et/ou la conversion d'un composé de formule générale (I) dans laquelle R' représente un atome d'hydrogène en un sel non toxique correspondant.

2. Un procédé selon la revendication 1, pour la préparation d'un composé de formule (I) selon lequel $R^2$ est un groupe de formule générale (i), ou un sel non toxique dudit composé.

3. Un procédé selon la revendication 2, pour la préparation d'un composé de formule (I) selon lequel les deux groupes $R^1$ sont des groupes méthyles, ou un sel non toxique de ce composé.

4. Un procédé selon la revendication 1 ou 3, pour la préparation d'un composé de formule (I) qui est :
l'acide 4-[2-(4-benzyloxy-2,3-diméthylbenzoylamino)phénoxy]butanoïque,
l'acide 4-[2-(4-(2-méthylbenzyloxy)-2,3-diméthylbenzoylamino]phénoxy]butanoïque,
l'acide 4-[2-[4-(3-méthylbenzyloxy)-2,3-diméthylbenzoylamino]-phénoxy]butanoïque,
l'acide 4-[2-[(4-méthylbenzyloxy)-2,3-diméthylbenzoylamino]phénoxy] butanoïque,
l'acide 4-[2-[4-(2,6-diméthylbenzyloxy)-2,3-diméthylbenzoylamino]-phénoxy]butanoïque,
l'acide 4-[2-[4-(4-éthylbenzyloxy)-2,3-diméthylbenzoylamino]-phénoxy]butanoïque,
l'acide 4-[2-[4-(4-propylbenzyloxy)-2,3-diméthylbenzoylamino]-phénoxy]butanoïque,
l'acide 4-[2-[4-(4-isopropylbenzyloxy)-2,3-diméthylbenzoylamino]-phénoxy]butanoïque,
l'acide 4-[2-[4-(4-isobutylbenzyloxy)-2,3-diméthylbenzoylamino]-phénoxy]butanoïque,
l'acide 4-[2-[4-(4-chlorobenzyloxy)-2,3-diméthylbenzoylamino]-phénoxy]butanoïque,
l'acide 4-[2-[4-(4-cyclobutylméthylbenzyloxy)-2,3-diméthylbenzoylamino]phénoxy]butanoïque,
l'acide 4-[2-[4-(2-phényléthoxy)-2,3-diméthylbenzoylamino]phénoxy]-butanoïque,
l'acide 4-[2-[4-(3-phénylpropoxy)-2,3-diméthylbenzoylamino]phénoxy] butanoïque,
l'acide 4-[2-[4-(4-phénylbutoxy)-2,3-diméthylbenzoylamino]phénoxy] butanoïque,
l'acide 4-[2-[4-(5-phénylpentyloxy)-2,3-diméthylbenzoylamino]-phénoxy]butanoïque,
l'acide 4-[2-[4-[1-(4-isobutylphényl)éthoxy]-2,3-diméthylbenzoylamino]phénoxy]butanoïque,
l'acide 4-[2-[4-(4-propylbenzyloxy)-2,3-diméthylbenzoylamino)-phénylthio]butanoïque,
l'acide 4-[2-[4-[1-(4-isobutylphényl)éthoxy]-2,3-diméthylbenzoylamino]phénylthio]butanoïque,
l'acide 4-[2-[4-(4-propylbenzyloxy)-2,3-diméthylbenzoylamino]-phénylsulfinyl]butanoïque ou
l'acide 4-[2-[4-[N-(4-trifluorométhylphénylméthyl)amino]-2,3-diméthylbenzoylamino]phénoxy]butanoïque ou un sel non toxique de l'acide.

5. Un procédé selon la revendication 2, pour la préparation d'un composé de formule (I), selon lequel les deux groupes $R^1$ représentent des atomes de chlore ou, ensemble avec les atomes de carbone du noyau benzène auxquels ils sont attachés, un noyau cyclopentane, cyclohexane ou benzène, ou un sel non toxique de ce composé.

6. Un procédé selon la revendication 1 ou 5, qui est
l'acide 4-[2-[4-(4-isobutylbenzyloxy)-2,3-dichlorobenzoylamino]-phénoxy]butanoïque,
l'acide 4-[2-(7-(4-isobutylbenzyloxy)indane-4-carbonylamino]-phénoxy]butanoïque,
l'acide 4-[2-[4-(4-isobutylbenzyloxy)-5,6,7,8-tétrahydronaphtalène-1-carbonylamino]phénoxy]butanoïque,
l'acide 4-[2-[4-[4-isobutylbenzyloxy)naphtalène-1-carbonylamino]-phénoxy]butanoïque,
l'acide 4-[2-[4-(4-isobutylbenzyloxy)-5,6,7,8-tétrahydronaphtalène-1-carbonylamino]phénylthio]-butanoïque ou un sel non toxique de l'acide.

7. Un procédé selon la revendication 1, pour la préparation d'un composé de formule (I) selon lequel $R^2$ est un groupe de formule générale (ii), ou un sel non toxique dudit composé.

8. Un procédé selon la revendication 1 ou 7, pour la préparation d'un composé de formule (I), qui est :
l'acide 4-[2-[4-[bis(4-propylphényl)méthoxy]-2,3-diméthylbenzoylamino]phénoxy]butanoïque,
l'acide 4-[2-(4-diphénylméthoxy)-2,3-diméthylbenzoylamino]phénoxy] butanoïque,
l'acide 4-[2-[4-[bis(4-propylphényl)méthylamino]-2,3-diméthylbenzoylamino]phénoxy]butanoïque ou
l'acide 4-[2-[4-[bis(4-propylphényl)méthylthio]-2,3-diméthylbenzoylamino]phénoxy]butanoïque ou un sel non toxique de l'acide.

9. Un procédé selon la revendication 1, pour la préparation d'un composé de formule (I) selon lequel $R^2$ est un groupe de formule générale (iii), ou un sel non toxique de ce composé.

10. Un procédé selon la revendication 1 ou 9, pour la préparation d'un composé de formule (I), qui est :
l'acide 4-[2-[4-[N,N-bis(4-propylphénylméthyl)amino]-2,3-diméthylbenzoylamino]phénoxy]butanoïque,
l'acide 4-[2-[4-[N,N-bis(4-trifluorométhylphénylméthyl)amino]-2,3-diméthylbenzoylamino]phénoxy]-

butanoïque ou
l'acide 4-[2-[4-[N-méthyl-N-(5,6,7,8-tétrahydronapht-1-yl]aminométhyl]-2,3-diméthylbenzoylamino]-phénoxy]butanoïque, ou un sel non toxique de l'acide.

**11.** Une composition cosmétique qui comprend un dérivé de l'acide benzoylaminophénoxybutanoïque de formule (I) tel que défini dans la revendication 1 ou un sel non toxique dudit dérivé.

**Patentansprüche**

**Patentansprüche für folgende Vertragsstaaten : AT, BE, CH, DE, FR, GB, IT, LI, LU, NL, SE**

**1.** Benzoylaminophenoxybuttersäurederivat der allgemeinen Formel:

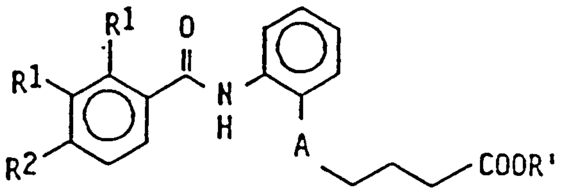

(I)

worin bedeuten:
R' ein Wasserstoffatom oder eine Alkylgruppe mit 1 bis 4 Kohlenstoffatom(en);
A ein Sauerstoff- oder Schwefelatom oder eine Sulfinyl-(SO)-Gruppe;
die beiden Gruppen $R^1$ gleichzeitig Methylgruppen oder Chloratome oder zusammen mit den Kohlenstoffatomen des Benzolrings, an denen sie hängen, einen Cyclopentan-, Cyclohexan- oder Benzolring;
$R^2$ eine Gruppe der allgemeinen Formeln:

(i)

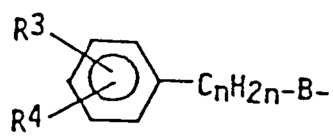

(ii)

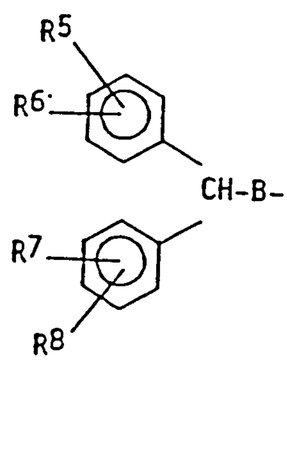

oder

(iii)

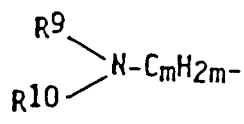

worin B für ein Sauerstoff- oder Schwefelatom oder eine Gruppe der allgemeinen Formel $NR^{11}$, mit $R^{11}$

gleich einem Wasserstoffatom oder einer Alkylgruppe mit 1 bis 4 Kohlenstoffatom(en) steht, $R^3$, $R^4$, $R^5$, $R^6$, $R^7$ und $R^8$ jeweils unabhängig voneinander ein Wasserstoffatom, eine Alkylgruppe mit 1 bis 4 Kohlenstoffatom(en), ein Halogenatom, eine Trifluormethylgruppe oder eine Cyclobutylmethylgruppe darstellen,
m = 0 oder 1,
n eine ganze Zahl von 1 bis 5 bedeutet und
$R^9$ und $R^{10}$, die gleich oder verschieden sein können, jeweils einem Wasserstoffatom, einer Alkylgruppe mit 1 bis 5 Kohlenstoffatom(en) oder einer Gruppe der allgemeinen Formeln

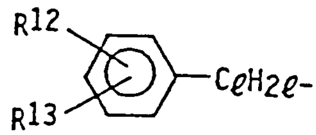

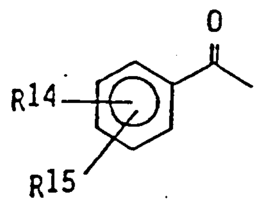

oder

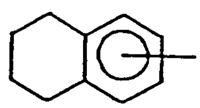

mit $R^{12}$, $R^{13}$, $R^{14}$ und $R^{15}$ jeweils, unabhängig voneinander, gleich einem Wasserstoffatom, einer Alkylgruppe mit 1 bis 4 Kohlenstoffatom(en), einem Halogenatom, einer Trifluormethylgruppe oder einer Cyclobutylmethylgruppe und
l gleich einer ganzen Zahl von 1 bis 4, entspricht,
wobei gilt, daß $R^9$ und $R^{10}$ nicht gleichzeitig für Wasserstoffatome stehen dürfen, oder ein nichttoxisches Salz desselben.

**2.** Verbindung nach Anspruch 1, worin $R^2$ für eine Gruppe der allgemeinen Formel (i) steht.

**3.** Verbindung nach Anspruch 2, worin beide Gruppen $R^1$ für Methylgruppen-stehen.

**4.** Verbindung nach Ansprüchen 1 oder 3, nämlich
4-[2-(4-Benzyloxy-2,3-dimethylbenzoylamino)-phenoxy]-buttersäure,
4-[2-[4-(2-Methylbenzyloxy)-2,3-dimethylbenzoylamino]-phenoxy]-buttersäure,
4-[2-[4-(3-Methylbenzyloxy)-2,3-dimethylbenzoylamino]-phenoxy]-buttersäure,
4-[2-[(4-Methylbenzyloxy)-2,3-dimethylbenzoylamino]-phenoxy]-buttersäure,
4-[2-[4-(2,6-Dimethylbenzyloxy)-2,3-dimethylbenzoylamino]-phenoxy]-buttersäure,
4-[2-[4-(4-Ethylbenzyloxy)-2,3-dimethylbenzoylamino]-phenoxy]-buttersäure,
4-[2-[4-(4-Propylbenzyloxy)-2,3-dimethylbenzoylamino]-phenoxy]-buttersäure,
4-[2-[4-(4-Isopropylbenzyloxy)-2,3-dimethylbenzoylamino]-phenoxy]-buttersäure,
4-[2-[4-(4-Isobutylbenzyloxy)-2,3-dimethylbenzoylamino]-phenoxy]-buttersäure,
4-[2-[4-(4-Chlorbenzyloxy)-2,3-dimethylbenzoylamino]-phenoxy]-buttersäure,
4-[2-[4-(4-Cyclobutylmethylbenzyloxy)-2,3-dimethylbenzoylamino]-phenoxy]-buttersäure,
4-[2-[4-(2-Phenylethoxy)-2,3-dimethylbenzoylamino]-phenoxy]-buttersäure,
4-[2-[4-(3-Phenylpropoxy)-2,3-dimethylbenzoylamino]-phenoxy]-buttersäure,
4-[2-[4-(4-Phenylbutoxy)-2,3-dimethylbenzoylamino]-phenoxy]-buttersäure,
4-[2-[4-(5-Phenylpentyloxy)-2,3-dimethylbenzoylamino]-phenoxy]-buttersäure,
4-[2-[4-[1-(4-Isobutylphenyl)-ethoxy]-2,3-dimethylbenzoylamino]-phenoxy]-buttersäure,
4-[2-[4-(4-Propylbenzyloxy)-2,3-dimethylbenzoylamino)-phenylthio]-buttersäure,

4-[2-[4-[1-(4-Isobutylphenyl)-ethoxy[-2,3-dimethylbenzoylamino]-phenylthio]-buttersäure,
4-[2-[4-(4-Propylbenzyloxy)-2,3-dimethylbenzoylamino]-phenylsulfinyl]-buttersäure oder
4-[2-[4-[N-(4-Trifluormethylphenylmethyl)-amino]-2,3-dimethylbenzoylamino]-phenoxy]-buttersäure
oder ein nicht-toxisches Salz derselben.

5. Verbindung nach Anspruch 2, worin die beiden Gruppen $R^1$ für Chloratome stehen oder zusammen mit den Kohlenstoffatomen des Benzolrings, an denen sie hängen, einen Cyclopentan-, Cyclohexan- oder Benzolring bilden.

6. Verbindung nach Ansprüchen 1 oder 5, nämlich
4-[2-[4-(4-Isobutylbenzyloxy)-2,3-dichlorbenzoylamino]-phenoxy]-buttersäure,
4-[2-(7-(4-Isobutylbenzyloxy)-indan-4-carbonylamino]-phenoxy]-buttersäure,
4-[2-[4-(4-Isobutylbenzyloxy)-5,6,7,8-tetrahydronaphthalin-1-carbonylamino]-phenoxy]-buttersäure,
4-[2-[4-(4-Isobutylbenzyloxy)-naphthalin-1-carbonylamino]-phenoxy]-buttersäure oder
4-[2-[4-(4-Isobutylbenzyloxy)-5,6,7,8-tetrahydronaphthalin-1-carbonylamino]-phenylthio]-buttersäure
oder ein nicht-toxisches Salz derselben.

7. Verbindung nach Anspruch 1, worin $R^2$ für eine Gruppe der allgemeinen Formel (ii) steht.

8. Verbindung nach Ansprüchen 1 oder 7, nämlich:
4-[2-[4-[Bis-(4-propylphenyl)-methoxy]-2,3-dimethylbenzoylamino]-phenoxy]-buttersäure,
4-[2-(4-Diphenylmethoxy)-2,3-dimethylbenzoylamino]-phenoxy]-buttersäure,
4-[2-[4-[Bis-(4-propylphenyl)-methylamino]-2,3-dimethylbenzoylamino]-phenoxy]-buttersäure oder
4-[2-[4-[Bis-(4-propylphenyl)-methylthio]-2,3-dimethylbenzoylamino]-phenoxy]-buttersäure
oder ein nicht-toxisches Salz derselben.

9. Verbindung nach Anspruch 1, worin $R^2$ für eine Gruppe der allgemeinen Formel (iii) steht.

10. Verbindung nach Ansprüchen 1 oder 9, nämlich:
4-[2-[4-[N,N-Bis-(4-propylphenylmethyl)-amino]-2,3-dimethylbenzoylamino]-phenoxy]-buttersäure,
4-[2-[4-[N,N-Bis-(4-trifluormethylphenylmethyl)amino]-2,3-dimethylbenzoylamino]-phenoxy]-buttersäure
oder
4-[2-[4-[N-Methyl-N-(5,6,7,8-tetrahydronaphth-1-yl)-aminomethyl]-2,3-dimethylbenzoylamino]-phenoxy]-buttersäure
oder ein nicht-toxisches Salz derselben.

11. Verfahren zur Herstellung eines Benzoylaminophenoxybuttersäurederivats nach Anspruch 1
(A) für die Herstellung von Verbindungen der allgemeinen Formel:

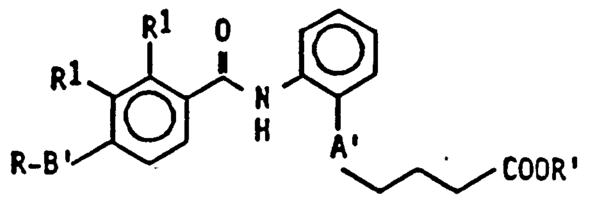

(Ia)

worin bedeuten:
A' ein Sauerstoff- oder Schwefelatom;
R eine Gruppe der allgemeinen Formeln

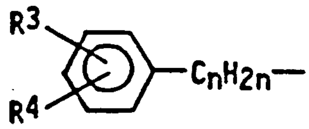 oder

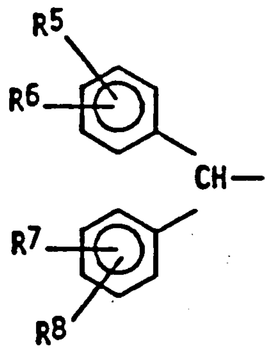

worin die verschiedenen Symbole die in Anspruch 1 angegebene Bedeutung besitzen, und
B' ein Sauerstoff- oder Schwefelatom und
worin die sonstigen Symbole die in Anspruch 1 angegebene Bedeutung besitzen, durch
Umsetzen einer Carbonsäure der allgemeinen Formel:

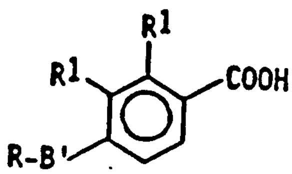

(II)

worin B' die zuvor angegebene Bedeutung besitzt und R und $R^1$ die in Anspruch 1 angegebene Bedeutung besitzen, oder eines aktivierten Derivats derselben mit einem Amin der allgemeinen Formel:

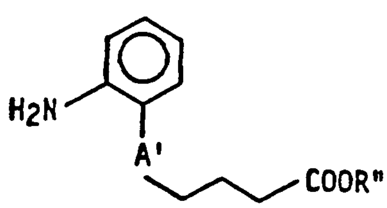

(III)

worin R" für eine Alkylgruppe mit 1 bis 4 Kohlenstoffatom(en) steht und A' die zuvor angegebene Bedeutung besitzt;
(B) für die Herstellung von Verbindungen der allgemeinen Formel:

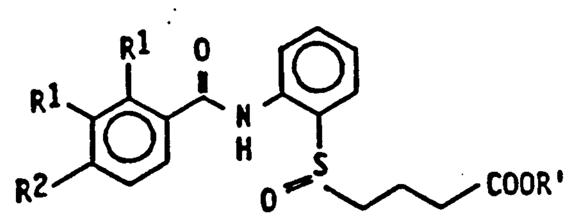

(Ib)

worin R', $R^1$ und $R^2$ die in Anspruch 1 angegebene Bedeutung besitzen, durch Oxidation einer Verbindung der allgemeinen Formel:

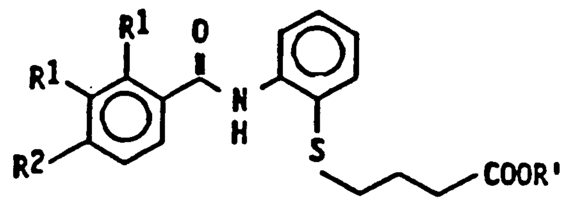

(Ib')

worin R', $R^1$ und $R^2$ die in Anspruch 1 angegebene Bedeutung besitzen, zur Umwandlung der Gruppe -S- in eine Gruppe -S(=O)- und
(C) für die Herstellung von Verbindungen der allgemeinen Formel:

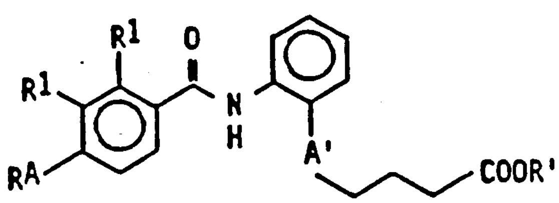

(Ic)

worin $R^A$ für eine Gruppe der allgemeinen Formeln:

(i')

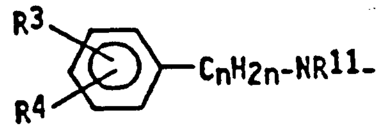

(ii')

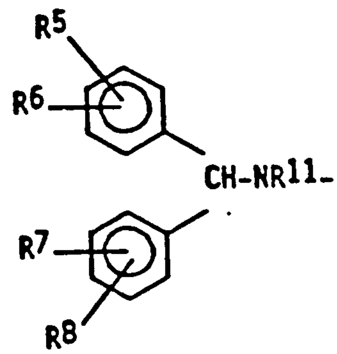

oder

(iii')

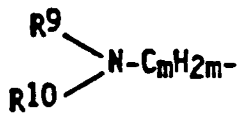

mit sämtlichen Symbolen in der in Anspruch 1 angegebenen Bedeutung, steht, $R^1$ und R' die in Anspruch 1 angegebene Bedeutung besitzen und A' der zuvor angegebenen Bedeutung entspricht, durch Umsetzen einer Verbindung der allgemeinen Formel:

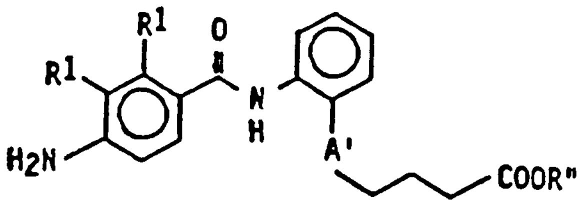

(IV)

worin $R^1$ die in Anspruch 1 angegebene Bedeutung besitzt und A' und R" der zuvor angegebenen Bedeutung entsprechen, mit einer Verbindung der allgemeinen Formeln:

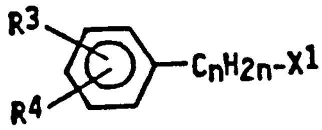

(V)

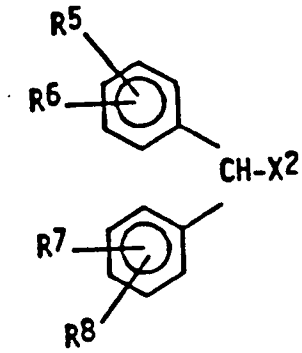

(VI)

$R^9$ - $X^3$ (VII)
$R^{10}$ - $X^4$ (VIII)

und/oder

$R^{11}$ - $X^5$ (IX)

worin $X^1$, $X^2$, $X^3$, $X^4$ und $X^5$ jeweils für ein Halogenatom, eine Tosylgruppe oder eine Mesylgruppe stehen und die sonstigen Symbole die in Anspruch 1 angegebene Bedeutung besitzen, oder durch Umsetzen einer Verbindung der allgemeinen Formel:

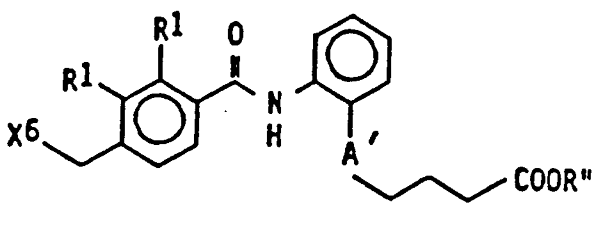

(X)

worin $X^5$ für ein Halogenatom, eine Tosylgruppe oder eine Mesylgruppe steht, $R^1$ die in Anspruch 1 angegebene Bedeutung besitzt und A' und R" der zuvor angegebenen Bedeutung entsprechen, mit einer Verbindung der allgemeinen Formel:

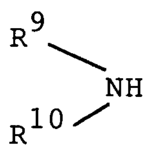

(XI)

worin $R^9$ und $R^{10}$ die in Anspruch 1 angegebene Bedeutung besitzen,
und gewünschtenfalls anschließend Hydrolysieren der jeweils erhaltenen Verbindung der allgemeinen Formel (I) zur Umwandlung der Gruppe -COOR' mit R' gleich einer Alkylgruppe mit 1 bis 4 Kohlenstoffatom(en) in eine Gruppe COOH und/oder
Umwandeln einer Verbindung der allgemeinen Formel (I) mit R' gleich einem Wasserstoffatom in ein

nicht-toxisches Salz derselben.

**12.** Arzneimittel mit einem Benzoylaminophenoxybuttersäurederivat nach Anspruch 1 oder einem nicht-toxischen Salz desselben in Verbindung mit einem pharmazeutisch akzeptablen Träger oder Überzug.

**13.** Benzoylaminophenoxybuttersäurederivat nach Anspruch 1 oder ein nicht-toxisches Salz desselben zur Verwendung bei der Herstellung eines Arzneimittels.

**14.** Verwendung eines Benzoylaminophenoxybuttersäurederivats nach Anspruch 1 oder eines nicht-toxischen Salzes desselben bei der Herstellung eines Arzneimittels zur Behandlung von Alopezie, Akne oder Prostatahypertrophie.

**15.** Kosmetische Zubereitung mit einem Benzoylaminophenoxybuttersäurederivat der allgemeinen Formel (I) oder einem nicht-toxischen Salz desselben.

**16.** Verwendung eines Benzoylaminophenoxybuttersäurederivats der allgemeinen Formel (I) oder eines nicht-toxischen Salzes desselben als kosmetisches Produkt bei der Behandlung von Akne oder Alkopezie.

**Patentansprüche für folgende Vertragsstaaten : ES, GR**

**1.** Verfahren zur Herstellung eines Benzoylaminophenoxybuttersäurederivats der allgemeinen Formel:

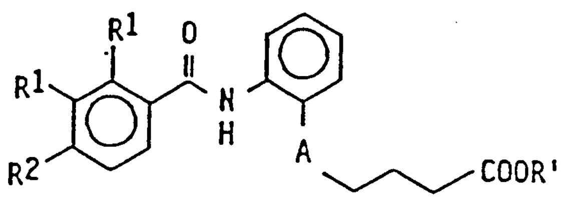

(I)

worin bedeuten:

R' ein Wasserstoffatom oder eine Alkylgruppe mit 1 bis 4 Kohlenstoffatom(en);

A ein Sauerstoff- oder Schwefelatom oder eine Sulfinyl-(SO)-Gruppe;

die beiden Gruppen $R^1$ gleichzeitig Methylgruppen oder Chloratome oder zusammen mit den Kohlenstoffatomen des Benzolrings, an denen sie hängen, einen Cyclopentan-, Cyclohexan- oder Benzolring;

$R^2$ eine Gruppe der allgemeinen Formeln:

(i) 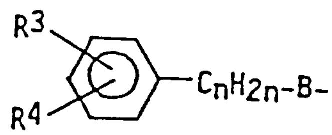

(ii) 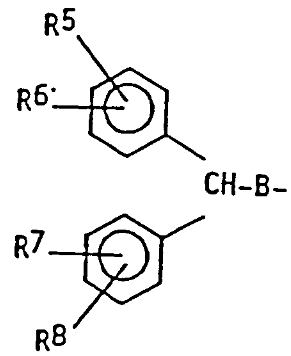 oder

(iii) 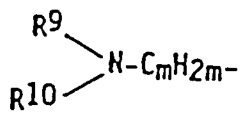

worin B für ein Sauerstoff- oder Schwefelatom oder eine Gruppe der allgemeinen Formel $NR^{11}$, mit $R^{11}$ gleich einem Wasserstoffatom oder einer Alkylgruppe mit 1 bis 4 Kohlenstoffatom(en) steht,
$R^3$, $R^4$, $R^5$, $R^6$, $R^7$ und $R^8$ jeweils unabhängig voneinander ein Wasserstoffatom, eine Alkylgruppe mit 1 bis 4 Kohlenstoffatom(en), ein Halogenatom, eine Trifluormethylgruppe oder eine Cyclobutylmethylgruppe darstellen,
m = 0 oder 1,
n eine ganze Zahl von 1 bis 5 bedeutet und
$R^9$ und $R^{10}$, die gleich oder verschieden sein können, jeweils einem Wasserstoffatom, einer Alkylgruppe mit 1 bis 5 Kohlenstoffatom(en) oder einer Gruppe der allgemeinen Formeln

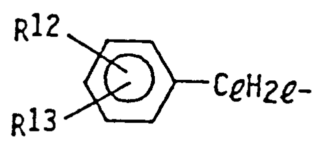

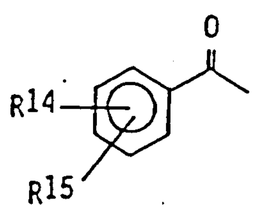

oder

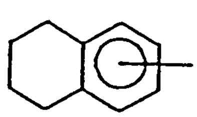

mit $R^{12}$, $R^{13}$, $R^{14}$ und $R^{15}$ jeweils, unabhängig voneinander, gleich einem Wasserstoffatom, einer Alkylgruppe mit 1 bis 4 Kohlenstoffatom(en), einem Halogenatom, einer Trifluormethylgruppe oder einer Cyclobutylmethylgruppe und
l gleich einer ganzen Zahl von 1 bis 4, entspricht,
wobei gilt, daß $R^9$ und $R^{10}$ nicht gleichzeitig für Wasserstoffatome stehen dürfen, oder eines nicht-toxischen Salzes desselben

(A) für die Herstellung von Verbindungen der allgemeinen Formel:

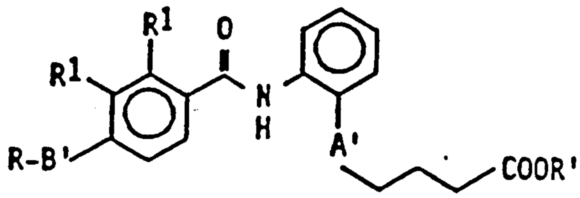

(Ia)

worin bedeuten:
A' ein Sauerstoff- oder Schwefelatom;
R eine Gruppe der allgemeinen Formeln

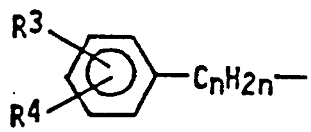

oder

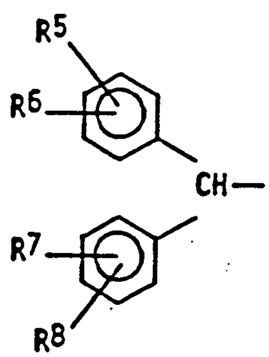

worin die verschiedenen Symbole die zuvor angegebene Bedeutung besitzen, und
B' ein Sauerstoff- oder Schwefelatom und
worin die sonstigen Symbole die zuvor angegebene Bedeutung besitzen, durch Umsetzen einer Carbonsäure der allgemeinen Formel:

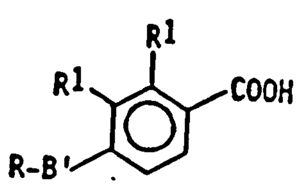

(II)

worin die verschiedenen Symbole die zuvor angegebene Bedeutung besitzen, oder eines aktivierten Derivats derselben mit einem Amin der allgemeinen Formel:

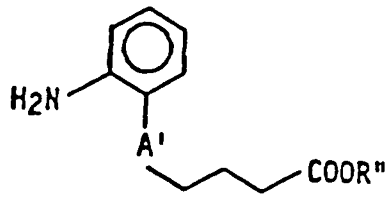

(III)

worin R" für eine Alkylgruppe mit 1 bis 4 Kohlenstoffatom(en) steht und A' die zuvor angegebene Bedeutung besitzt;
(B) für die Herstellung von Verbindungen der allgemeinen Formel:

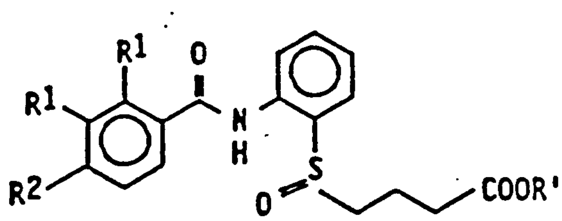

(Ib)

worin R', $R^1$ und $R^2$ die zuvor angegebene Bedeutung besitzen, durch Oxidation einer Verbindung der allgemeinen Formel:

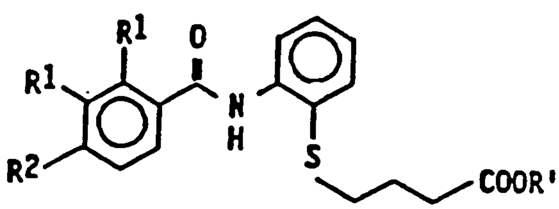

(Ib')

worin R', $R^1$ und $R^2$ die zuvor angegebene Bedeutung besitzen, zur Umwandlung der Gruppe -S- in eine Gruppe -S(=O)- und
(C) für die Herstellung von Verbindungen der allgemeinen Formel:

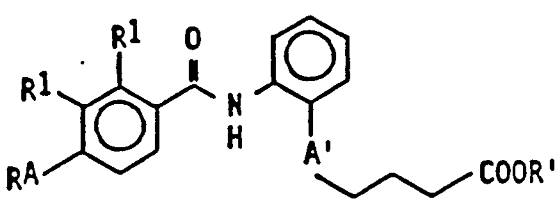

(Ic)

worin $R^A$ für eine Gruppe der allgemeinen Formeln:

(i')

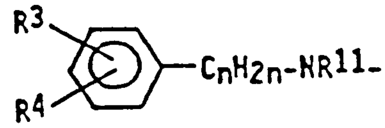

(ii')

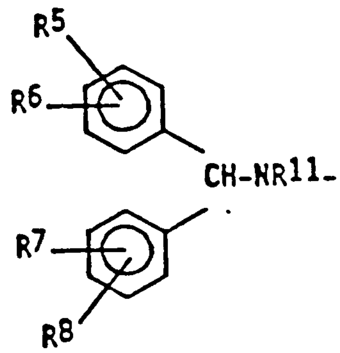

oder

(iii')

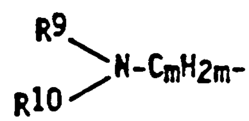

worin sämtliche Symbole die angegebene Bedeutung besitzen und $R^1$, R' and A' der zuvor angegebenen Bedeutung entsprechen, steht, durch Umsetzen einer Verbindung der allgemeinen Formel:

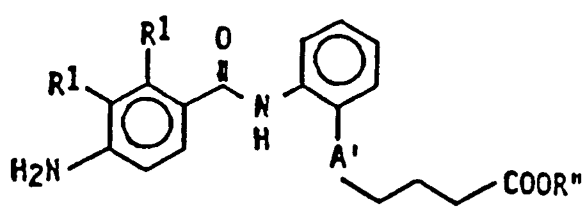

(IV)

worin $R^1$, A' und R" der zuvor angegebenen Bedeutung entsprechen, mit einer Verbindung der allgemeinen Formeln:

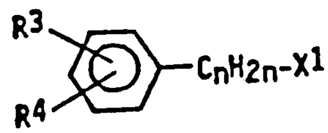

(V)

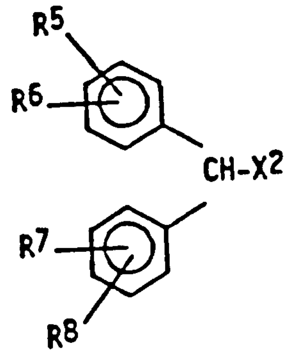

(VI)

$R^9 - X^3$ (VII)
$R^{10} - X^4$ (VIII)

und/oder

$R^{11} - X^5$ (IX)

worin $X^1$, $X^2$, $X^3$, $X^4$ und $X^5$ jeweils für ein Halogenatom, eine Tosylgruppe oder eine Mesylgruppe stehen und die sonstigen Symbole der zuvor angegebenen Bedeutung entsprechen, oder durch Umsetzen einer Verbindung der allgemeinen Formel:

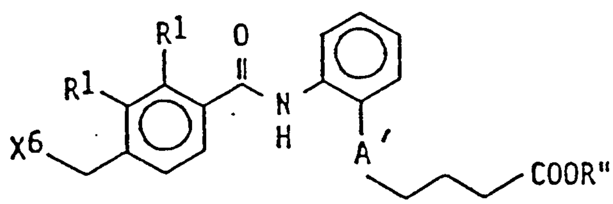

(X)

worin $X^5$ für ein Halogenatom, eine Tosylgruppe oder eine Mesylgruppe steht und $R^1$, A' und R" der zuvor angegebenen Bedeutung entsprechen, mit einer Verbindung der allgemeinen Formel:

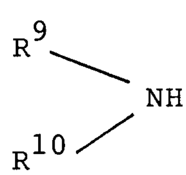

(XI)

worin $R^9$ und $R^{10}$ die zuvor angegebene Bedeutung besitzen,
und gewünschtenfalls anschließend Hydrolysieren der jeweils erhaltenen Verbindung der allgemeinen Formel (I) zur Umwandlung der Gruppe -COOR' mit R' gleich einer Alkylgruppe mit 1 bis 4 Kohlenstoffatom(en) in eine Gruppe COOH und/oder
Umwandeln einer Verbindung der allgemeinen Formel (I) mit R' gleich einem Wasserstoffatom in ein

nicht-toxisches Salz derselben.

2. Verfahren nach Anspruch 1 zur Herstellung einer Verbindung der Formel (I), worin $R^2$ für eine Gruppe der allgemeinen Formel (i) steht, oder eines nicht-toxischen Salzes derselben.

3. Verfahren nach Anspruch 2 zur Herstellung einer Verbindung der Formel (I), worin beide Gruppen $R^1$ für Methylgruppen stehen, oder eines nicht-toxischen Salzes desselben.

4. Verfahren nach Anspruch 1 oder 3 zur Herstellung einer Verbindung der Formel (I), nämlich
4-[2-(4-Benzyloxy-2,3-dimethylbenzoylamino)-phenoxy]-buttersäure,
4-[2-[4-(2-Methylbenzyloxy)-2,3-dimethylbenzoylamino]phenoxy]-buttersäure,
4-[2-[4-(3-Methylbenzyloxy)-2,3-dimethylbenzoylamino]phenoxy]-buttersäure,
4-[2-[(4-Methylbenzyloxy)-2,3-dimethylbenzoylamino]phenoxy]-buttersäure,
4-[2-[4-(2,6-Dimethylbenzyloxy)-2,3-dimethylbenzoylamino]phenoxy]-buttersäure,
4-[2-[4-(4-Ethylbenzyloxy)-2,3-dimethylbenzoylamino]phenoxy]-buttersäure,
4-[2-[4-(4-Propylbenzyloxy)-2,3-dimethylbenzoylamino]phenoxy]-buttersäure,
4-[2-[4-(4-Isopropylbenzyloxy)-2,3-dimethylbenzoylamino]phenoxy]-buttersäure,
4-[2-[4-(4-Isobutylbenzyloxy)-2,3-dimethylbenzoylamino]phenoxy]-buttersäure,
4-[2-[4-(4-Chlorbenzyloxy)-2,3-dimethylbenzoylamino]phenoxy]-buttersäure,
4-[2-[4-(4-Cyclobutylmethylbenzyloxy)-2,3-dimethylbenzoylamino]-phenoxy]-buttersäure,
4-[2-[4-(2-Phenylethoxy)-2,3-dimethylbenzoylamino]phenoxy]-buttersäure,
4-[2-[4-(3-Phenylpropoxy)-2,3-dimethylbenzoylamino]phenoxy]-buttersäure,
4-[2-[4-(4-Phenylbutoxy)-2,3-dimethylbenzoylamino]phenoxy]-buttersäure,
4-[2-[4-(5-Phenylpentyloxy)-2,3-dimethylbenzoylamino]-phenoxy]-buttersäure,
4-[2-[4-[1-(4-Isobutylphenyl)-ethoxy]-2,3-dimethylbenzoylamino]-phenoxy]-buttersäure,
4-[2-[4-(4-Propylbenzyloxy)-2,3-dimethylbenzoylamino)-phenylthio]-buttersäure,
4-[2-[4-[1-(4-Isobutylphenyl)-ethoxy[-2,3-dimethylbenzoylamino]-phenylthio]-buttersäure,
4-[2-[4-(4-Propylbenzyloxy)-2,3-dimethylbenzoylamino]-phenylsulfinyl]-buttersäure oder
4-[2-[4-[N-(4-Trifluormethylphenylmethyl)-amino]-2,3-dimethylbenzoylamino]-phenoxy]-buttersäure,
oder eines nicht-toxischen Salzes desselben.

5. Verfahren nach Anspruch 2 zur Herstellung einer Verbindung der Formel (I), worin die beiden Gruppen $R^1$ für Chloratome stehen oder zusammen mit den Kohlenstoffatomen des Benzolrings, an denen sie hängen, einen Cyclopentan-, Cyclohexan- oder Benzolring bilden, oder eines nicht-toxischen Salzes desselben.

6. Verfahren nach Ansprüchen 1 oder 5 zur Herstellung einer Verbindung der Formel (I), nämlich
4-[2-[4-(4-Isobutylbenzyloxy)-2,3-dichlorbenzoylamino]-phenoxy]-buttersäure,
4-[2-(7-(4-Isobutylbenzyloxy)-indan-4-carbonylamino]-phenoxy]-buttersäure,
4-[2-[4-(4-Isobutylbenzyloxy)-5,6,7,8-tetrahydronaphthalin-1-carbonylamino]-phenoxy]-buttersäure,
4-[2-[4-(4-Isobutylbenzyloxy)-naphthalin-1-carbonylamino]-phenoxy]-buttersäure oder
4-[2-[4-(4-Isobutylbenzyloxy)-5,6,7,8-tetrahydronaphthalin-1-carbonylamino]-phenylthio]-buttersäure,
oder eines nicht-toxischen Salzes derselben.

7. Verfahren nach Anspruch 1 zur Herstellung einer Verbindung der Formel (I), worin $R^2$ für eine Gruppe der allgemeinen Formel (ii) steht, oder eines nicht-toxischen Salzes derselben.

8. Verfahren nach Ansprüchen 1 oder 7 zur Herstellung einer Verbindung der Formel (I), nämlich
4-[2-[4-[Bis-(4-propylphenyl)-methoxy]-2,3-dimethylbenzoylamino]-phenoxy]-buttersäure,
4-[2-(4-Diphenylmethoxy)-2,3-dimethylbenzoylamino]-phenoxy]-buttersäure,
4-[2-[4-[Bis-(4-propylphenyl)-methylamino]-2,3-dimethylbenzoylamino]-phenoxy]-buttersäure oder
4-[2-[4-[Bis-(4-propylphenyl)-methylthio]-2,3-dimethylbenzoylamino]-phenoxy]-buttersäure,
oder eines nicht-toxischen Salzes derselben.

9. Verfahren nach Anspruch 1 zur Herstellung einer Verbindung der Formel (I), worin $R^2$ für eine Gruppe der allgemeinen Formel (iii) steht, oder eines nicht-toxischen Salzes derselben.

10. Verfahren nach Ansprüchen 1 oder 9 zur Herstellung einer Verbindung der Formel (I), nämlich

4-[2-[4-[N,N-Bis-(4-propylphenylmethyl)-amino]-2,3-dimethylbenzoylamino]-phenoxy]-buttersäure,
4-[2-[4-[N,N-Bis-(4-trifluormethylphenylmethyl)amino]-2,3-dimethylbenzoylamino]-phenoxy]-buttersäure oder
4-[2-[4-[N-Methyl-N-(5,6,7,8-tetrahydronaphth-1-yl)-aminomethyl]-2,3-dimethylbenzoylamino]-phenoxy]-buttersäure,
oder eines nicht-toxischen Salzes derselben.

**11.** Kosmetische Zubereitung mit einem Benzoylaminophenoxybuttersäurederivat der Formel (I) in der Definition gemäß Anspruch 1 oder einem nicht-toxischen Salz desselben.